# EUROPEAN PATENT APPLICATION

(11) **EP 2 067 858 A1**
(43) Date of publication of application: **10.06.2009**
(21) Application number: 07380344.7
(22) Date of filing: 07.12.2007
(51) Int. Cl.: C12N 15/00, A01K 67/00, A61K 49/00, C07K 14/00

(54) **Animal models for neurodegenerative diseases**

(71) Applicant: UNIVERSIDAD DE SEVILLA, 41001 Sevilla (ES)
(72) Inventor: Pascual Bravo, Alberto Instituto de Biomedicina, Avda. Manuel Siurot S/N 41013 Sevilla (ES); Hidalgo Figueroa, María Instituto de Biomedicina, Avda. Manuel Siurot S/N 41013 Sevilla (ES); Gómez Díaz, Raquel Instituto de Biomedicina, Avda. Manuel Siurot S/N 41013 Sevilla (ES); López-Barneo, José Instituto de Biomedicina, Avda. Manuel Siurot S/N 41013 Sevilla (ES); Piruat Palomo, José Ignacio Instituto de Biomedicina, Avda. Manuel Siurot S/N 41013 Sevilla (ES); Pintado Sanjuan, Carmelo Oscar Instituto de Biomedicina, Avda. Manuel Siurot S/N 41013 Sevilla (ES)
(74) Representative: ABG Patentes, S.L.

(57) **Abstract**

The invention provides a non-human animal model of Parkinson's disease based on animals which carry a modified version of the GDNF gene which comprises recombinase target sites flanking an essential part of said gene and a transgene which encodes for a recombinase specific for the target sites in the GDNF gene and which can be activated at will by administering a given compound to the animal. Activation of the recombinase in adult animals leads to a progressive catecholaminergic neuronal death. The invention also provides the use of said animals for the study of the pathogenesis of Parkinson's disease and for the identification of GDNF targets (which preserve the neurons from degeneration) as well as GDNF agonists and activators.

## Description

### FIELD OF THE INVENTION

The invention relates to the field of neurodegenerative diseases. In particular, the invention relates to genetically modified animals carrying a conditional mutation in a gene which, when activated, leads to a symptoms reminiscent of certain neurodegenerative diseases. These animals are suitable for the identification of compounds which might be suitable for the treatment and prevention of said diseases.

### BACKGROUND OF THE INVENTION

Parkinson's disease (also known as Parkinson disease or PD) is a degenerative disorder of the central nervous system that often impairs the sufferer's motor skills and speech.

Parkinson's disease belongs to a group of conditions called movement disorders. It is characterized by muscle rigidity, tremor, a slowing of physical movement (bradykinesia) and, in extreme cases, a loss of physical movement (akinesia). The primary symptoms are the results of decreased stimulation of the motor cortex by the basal ganglia, normally caused by the insufficient formation and action of dopamine, which is produced in the dopaminergic neurons of the brain. Secondary symptoms may include high level cognitive dysfunction and subtle language problems. PD is both chronic and progressive. PD is the most common cause of parkinsonism, a group of similar symptoms. PD is also called "primary parkinsonism" or "idiopathic PD" (having no known cause). While most forms of parkinsonism are idiopathic, there are some cases where the symptoms may result from toxicity, drugs, genetic mutation, head trauma, or other medical disorders.

The symptoms of Parkinson's disease result from the loss of pigmented dopamine-secreting (dopaminergic) cells, secreted by the same cells, in the pars compacta region of the substantia nigra (literally "black substance"). These neurons project to the striatum and their loss leads to alterations in the activity of the neural circuits within the basal ganglia that regulate movement, in essence an inhibition of the direct pathway and excitation of the indirect pathway. The direct pathway facilitates movement and the indirect pathway inhibits movement, thus the loss of these cells leads to a hypokinetic movement disorder. The lack of dopamine results in increased inhibition of the ventral lateral nucleus of the thalamus, which sends excitatory projections to the motor cortex, thus leading to hypokinesia. There are four major dopamine pathways in the brain; the nigrostriatal pathway, referred to above, mediates movement and is the most conspicuously affected in early Parkinson's disease. The other pathways are the mesocortical, the mesolimbic, and the tuberoinfundibular. These pathways are associated with, respectively: volition and emotional responsiveness; desire, initiative, and reward; and sensory processes and maternal behaviour. Disruption of dopamine along the non-striatal pathways likely explains much of the neuropsychiatric pathology associated with Parkinson's disease.

Typically, PD has been studied using animal models obtained using substances such as 6-hydroxydopamine, paraquat, MPTP, 6-OHDA, rotenone, and epoxomicin which are captured and metabolized by dopaminergic neurons resulting in toxic products which destroy the cells. However, these methods have the disadvantage of the relatively low specificity of the substance which can be captured not only by dopaminergic neurons but also by noradrenergic neurons so that the effects observed may not always corresponds to the defects appearing in PD. Moreover, these animal models suffer from high variability due to the short half-lives of the drugs used and to the administration process.

More recently, gene-based animal models for PD have been developed which seem suitable to test neuroprotection in PD since they present replicable lesions, a predictable pattern of neurodegeneration and a well-characterized behaviour, biochemistry and morphology to assist in the understanding of induced changes.

In a first type of gene-based animal models, the animals are modified so that they either lack a gene which is essential for the development of dopaminergic neurons or they are modified so that they express a gene which is toxic for said cells. For instance, it is known from EP1688036 an animal model for Parkinson's disease containing a transgene comprising the coding region of α-synuclein in neurons operably linked to a the neuron-specific tyrosine hydroxylase promoter. US2003022876 describes an animal model for PD obtained by inserting the TNF gene under the control of the Engrailed-1 gene promoter, which leads to the over-expression of TNF in the *substantia nigra* and subsequent cellular degeneration. WO0116176 describes an animal model obtained by homologous recombination or PD which carries an inactive version of the Parkin-2 gene.

However, these methods imply the expression of the protein or of the defect in all tissues wherein the promoter which control the expression of said protein is activated, so that it can be difficult to establish differences between effects due to the toxicity on the cells from defects associated to development in the absence of a protein or in the presence of the toxic gene. In fact, if the gene which is inactivated is essential for embryonic development, it might be impossible to obtain adult animals carrying a homozygous deletion in said gene.

Therefore, it is preferred to use methods which allow the induction of the lesion in a time-controlled manner. Methods which allow the induction of the lesion in a time-specific manner have been described in the art. For instance, EP 18008070 describes an animal model for PD obtained by stereotaxic application into the CNS of a viral vector encoding a dominant negative variant of the IGF-1 receptor. Ogata et al (J.Neurovirol., 1997, 3:141-147) have described an animal model which resembles some of the findings of PD obtained by infection with Japanese encephalitis virus under particular circumstances.

However, all these methods involve the use of neurotropic viruses which may result in undesired side effects due to the neurotoxicity caused by the virus infection rather than to the genetic modification of the infected cells. In this regard, the use of the conditional targeting of a gene of interest by the use of recombinases may provide an advantageous alternative to the use of virus when a genetic lesion is to be introduced in a time-controlled manner. Conditional targeting has been widely used for the study of the nervous system, but only in few instances has led to the generation of an animal model for a neurological disease. For instance, it is known from EP1116790 to obtain an animal model for different neurodegenerative diseases, including spinal muscular atrophy, by recombinase-mediated excision of an essential region of the survival motor neuron gene (SMN) using a recombinase under the control of a neuron-specific enolase promoter. Kwon et al (Nat.Genet., 2001, 29:404-411) describe an animal model of Lhermitte-Duclos disease which carry a conditional deletion in the PTEN gene.

Glial cell-derived neurotrophic factor (GDNF) has been suggested to be capable of preventing dopaminergic neuron degeneration in PD. For instance, it is known from Chen et al. (Parkinson Relat. Disord., 2003, 10:1-7) and Lapchak et al (Brain Res., 1997, 777:153-160) that adenoviral-mediated gene transfer in a rat model of PD may show some protective effects. Moreover, WO0390689 describe a protective effect of neural stem cells which produce GDNF promote neurite outgrowth and body size on cultured dopaminergic neurons. However, the potential role of GDNF in ameliorating the symptoms of PD has been recently disputed (Do Thi, N. et al. 2007, Gene Ther. 14:441-450). These results have led to the idea that an animal model for PD could be obtained by introducing a conditional knock-out in a gene involved in GDNF signaling. It is however known from Jain, S. et al. (J. Neuroscience, 2006, 26:11230-11238) that mice carrying a conditional knock-out mouse of the RET tyrosine kinase gene, a gene acting in downstream of GDNF signaling, do not show any apparent loss of dopaminergic neurons or in the sensory and motor behavior of the animals. Additionally Kramer et al. (PLOS Biol. 2007, 5:e39) have described mice carrying conditional deletions in the GDNF receptor (Ret) but were only capable of observing late and partial destruction of the nigrostriatal pathway without observing any defects in the ventral tegmental area and in the locus coeruleus.

Therefore, there remains a need in the art of an animal model for PD which can be specifically turned in a time-controlled fashion and which overcomes the disadvantages of all other systems known in the art.

### SUMMARY OF THE INVENTION

In a first aspect, the invention relates to a DNA construct comprising at least a region of the GDNF gene flanked by recombinase target sites, wherein said region of the GDNF gene is necessary for the expression of a functional GDNF protein and wherein said recombinase target sites are oriented so that the intervening sequence is deleted in the presence of the recombinase.

The invention also relates to vectors and host cells comprising the construct of the invention.

In a further aspect, the invention relates to a cell which results from the homologous recombination of the targeting construct of the invention with the GDNF locus.

In another aspect, the invention relates to a method for the generation of a transgenic non-human animal, comprising the steps of propagating the cell wherein at least one of the GDNF loci has been modified by recombination with the targeting construct and developing from said cell a viable organism, wherein said cell is non-human. In another aspect, the invention relates to a non-human animal obtainable by the method according to said aspect.

In yet another aspect, the invention relates to a method for the generation of a transgenic non-human animal which comprises the steps of breeding animals carrying a modified GDNF locus and selecting those animals wherein both loci of the GDNF are modified. The invention also provides animals obtained by said method.

In another aspect, the invention relates to a method for obtaining a cell or a non-human animal carrying an inactive GDNF gene comprising the steps of
(a) contacting a cell carrying a modified GDNF locus or an animal carrying at least a modified GDNF locus with a compound which is capable of inducing the expression of a recombinase specific for the target sites present in the GDNF and
(b) selecting those cells or animals wherein the recombinase has catalysed the excision of the functional part of the GDNF locus.

In another aspect, the invention provides a cell or a non-human animal obtainable by a said method.

In another aspect, the invention provides a method for the generation of a conditional knock-out cell in the GDNF gene comprising the steps of:
(a) introducing a nucleic acid molecule comprising the DNA construct of the invention into a cell which expresses a recombinase whose expression and/or activity can be regulated by an external signal and
(b) selecting cells which wherein the DNA construct introduced in step (a) has recombined with the GDNF locus.

In yet another aspect, the invention relates to a method for the generation of a conditional knock-out cell in the GDNF gene, comprising the steps of
(a) introducing into a cell a first nucleic acid molecule comprising the DNA construct of the invention,
(b) selecting those cells wherein the DNA construct has recombined with the GDNF locus introducing into the cells selected in step (b) a second nucleic acid molecule coding for a recombinase whose expression and/or activity can be regulated by an external signal and
(c) selecting those cells which have integrated into their genome the second nucleic acid molecule.

In another aspect, the invention relates to a method for the generation of a conditional knock-out cell in the GDNF gene, comprising the steps of:
(a) introducing into a cell a first nucleic acid molecule comprising the DNA construct of the invention and a second nucleic acid molecule coding for a recombinase whose expression and/or activity can be regulated by an external signal and
(b) selecting those cells wherein the DNA construct of the first nucleic acid molecule has recombined with the GDNF locus and which have incorporated into their genome said second nucleic acid molecules.

In another aspect, the invention relates to a cell generated by the methods as defined above.

In yet another aspect, the invention relates to a method of obtaining a cell carrying an inactive GDNF gene comprising the steps of
(a) contacting a cell carrying a conditional knock-out in the GDNF gene with a compound which is capable of inducing the expression and/or activity of the recombinase and
(b) selecting those cells wherein the recombinase has catalysed the excision of a functional part of the GDNF locus.

In yet another aspect, the invention relates to a method for the generation of a transgenic non-human animal, comprising the steps of
(a) introducing into a cell a nucleic acid molecule comprising a DNA construct of the invention wherein said cell is a embryonic stem cell or an adult stem non-human cell
(b) selecting those cells wherein the DNA construct has recombined with the GDNF locus,
(c) developing from said cell a viable non-human animal,
(d) breeding the non-human animals obtained in step (c) with a strain from the same non-human species which contains into their genome a second nucleic DNA construct coding for a recombinase whose expression and/or activity can be regulated by an external signal and
(e) selecting those animals from the offspring which contain both the first nucleic acid molecule and the second nucleic acid molecule.

In another aspect, the invention relates to a method for the generation of a transgenic non-human animal, comprising the steps of
(a) introducing into a cell a first nucleic acid molecule comprising the DNA construct of the invention,
(b) selecting those cells wherein the DNA construct has recombined with the GDNF locus,
(c) introducing into the cells selected in step (b) a second nucleic acid molecule comprising the DNA construct of a second nucleic acid coding for a recombinase whose expression and/or activity can be regulated by an external signal,
(d) selecting cells which have integrated the second nucleic acid molecule and
(e) developing from said cell a viable non-human animal.

In yet another aspect, the invention relates to a transgenic non-human animal produced by the methods defined above.

In another aspect, the invention relates to a method for the generation of a transgenic non-human animal, comprising the steps of breeding animals of the invention and selecting those animals wherein both loci of the GDNF gene have recombined with the targeting construct.

In another aspect, the invention relates to a method for obtaining a non-human animal deficient in at least one GDNF allele which comprises contacting an animal carrying a modified GDNF locus comprising recombinase target sites flanking an essential region of the GDNF gene with a compound which is able to induce the expression or activity of the recombinase so that the recombinase-mediated excision of a functional part of the GDNF locus takes place.

In a further aspect, the invention relates to a non-human animal obtainable by the methods of the invention, as well as to organs, tissues or cells isolated from said non-human animal which is a non-embryonic animal.

In yet another aspect, the invention relates to a method for the identification of compounds suitable for the treatment and/or prevention of a neurodegenerative disease comprising
(a) contacting a non-human animal carrying a deletion in at least a GDNF gene with a test compound and
(b) selecting those compounds which prevent or ameliorate neuronal degeneration

In another aspect, the invention relates to the use of a non-human animal of the invention for the study of the mechanisms involved in neuronal degeneration induced by GDNF deficiency

In yet another aspect, the invention relates to a method for the generation of a conditional knock-out cell in the GDNF gene comprising the steps of:
(a) introducing a nucleic acid molecule comprising the DNA construct of the invention into a cell which contains within its genome a gene coding for a recombinase operably linked to a tissue-specific promoter and
(b) selecting cells wherein the DNA construct has recombined with the GDNF locus.

In another aspect, the invention relates to a method for the generation of a conditional knock-out cell in the GDNF gene, comprising the steps of:
(a) introducing into a cell a first nucleic acid molecule comprising the DNA construct of the invention,
(b) selecting cells wherein the DNA construct has recombined with the GDNF locus,
(c) introducing into the cells selected in step (b) a second nucleic acid molecule comprising a gene coding for a recombinase which is operably linked to a tissue specific promoter and
(d) selecting cells which have integrated into their genome the second nucleic acid molecule

In another aspect, the invention relates to a method for the generation of a conditional knock-out cell in the GDNF gene, comprising the steps of:
(a) introducing into a cell a first nucleic acid molecule comprising the DNA construct of the invention and a second nucleic acid molecule coding for a recombinase which is operably linked to a tissue-specific promoter and
(b) selecting those cells wherein the DNA construct has recombined with the GDNF locus and which have incorporated into their genome said second nucleic acid molecules.

In another aspect, the invention relates to a cell generated by the method of as defined previously.

In another aspect, the invention relates to a method for the generation of transgenic non-human animal, comprising the steps of propagating the cell of the invention and developing from said cell a viable organism, wherein said cell is non-human.

In another aspect, the invention relates to a method for the generation of a transgenic non-human animal, comprising the steps of
(a) introducing into a cell a first nucleic acid molecule comprising the DNA construct of the invention wherein said cell is a embryonic stem cell or an adult stem non-human cell
(b) selecting those cells wherein the DNA construct has recombined with the GDNF locus,
(c) developing from said cell a viable non-human animal,
(d) breeding the non-human animals obtained in step (c) with a strain from the same non-human species which contains into their genome a second nucleic DNA construct coding for a recombinase which is operably linked to a tissue-specific promoter and
(e) selecting those animals from the offspring which contain both the first nucleic acid molecule and the second nucleic acid molecule.

In another aspect, the invention relates to a transgenic non-human animal produced by the method defined above as well as to organs, tissues or cells isolated from said non-human animal.

In yet another aspect, the invention relates to the use of a non-human animal as defined above for the identification of compounds which promote the GDNF mRNA up-regulation in response to a down-regulation of GDNF in the neighbouring cells, for the identification of compounds which prevent the up-regulation of GDNF mRNA and for studying of the role of GDNF in embryonic development.

### BRIEF DESCRIPTION OF THE FIGURES

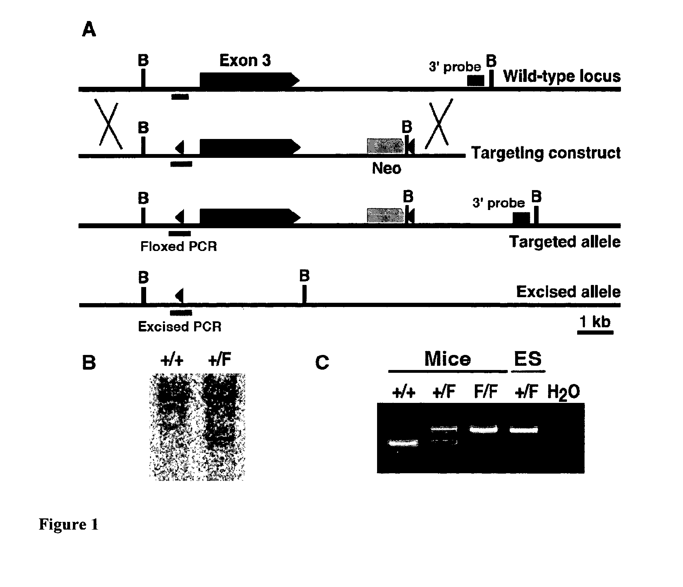

### Figure 1. GDNF Conditional Mice Generation

(A) Generation of a floxed allele of the GDNF gene. GDNF wild-type locus, targeting construct, floxed and excised alleles are shown. B, *Bam*HI. Southern blot probe and genotyping PCRs are indicated. Gray rectangles represent Neo marker and triangles loxP sequences. (B) ES cells clones showing correct targeting at the GDNF locus by Southern blotting. *Bam*HI digested DNA was hybridized with the 3' probe indicated in (A). An additional band is present in *GDNF*^{+/*F*} ES cells (+/F). (C) Floxed PCR used for genotyping of *GNDF*^{+/+} (+/+), *GDNF*^{+/*F*} (+/F), and *GDNF*^{*F*/*F*} (F/F) mice are shown. The ES *GDNF*^{+/*F*} cell line is shown as a control (+/F). *n* = 3-6 brains per group.

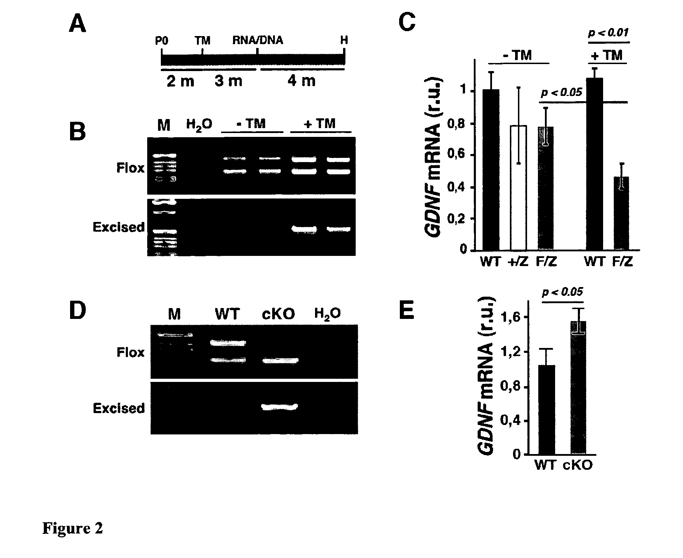

### Figure 2. Molecular characterization of conditional animal models

(A) Experimental protocol followed in the analysis of tamoxifen (TM)-treated animals. Time points (months) of TM injection and brain harvesting for RNA/DNA and histological analyses (H) are indicated. (B) PCR used to demonstrate the presence of the excised GDNF allele in striatum after TM treatment. Cre-Esr1 panel shows *GDNF*^{*Z*/}*^{F}, Cre-Esr1* animals without (-TM) and with (+TM) TM treatment. GFAP-Cre panel shows *GDNF* E3 excision in *GDNF*^{*F*/+} (WT) and *GDNF*^{*F*/*Z*}, *GFAP-Cre* (cKO) mice. (C) Relative levels of *GDNF* mRNA determined by quantitative RT-PCR. (Analysis of Cre-Esr1 mice. WT: *GDNF*^{*F*/+}*;* +/Z: *GDNF*^{+/*Z*}, *Cre-Esr;* F/Z: *GDNF*^{*F*/}*^{Z}, Cre-Esr1.* Analysis of GFAP-Cre mice. WT: *GDNF*^{*F*/+}*;* F/Z: *GDNF*^{*F*/}*^{Z}, GFAP-Cre*). Means ± S.E.M.; *, *p* < 0.05; **, *p* < 0.01 (Student t-test). *n* = 3-6 brains per group.

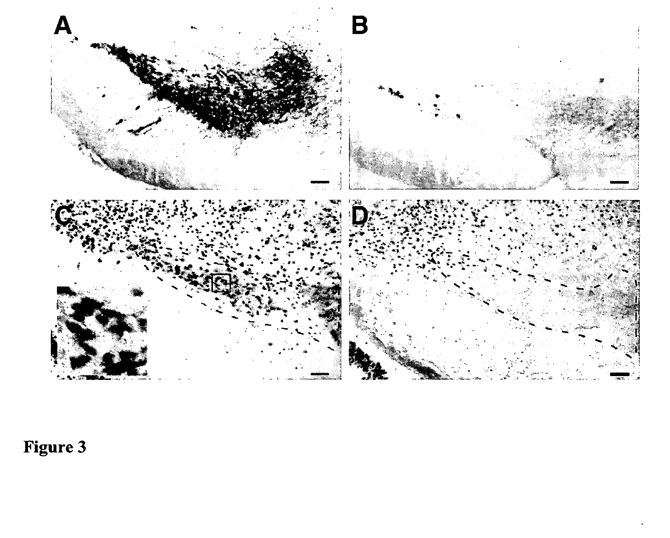

### Figure 3. SN and VTA Neuronal Death After Ablation of GDNF in Adult Brain

(A-B) Mesencephalic brain slices stained with an antibody against TH. *GDNF*^{*Z*/}*^{F}; Cre-Esr1*/+ without (A) and with (B) TM treatment. (C-D) Double immunostaining for NeuN (nuclear) and TH (cytoplasmic). The stippled line surrounds the substantia nigra (SN) and ventral tegmental area (VTA). Inset in C represents an amplification of the indicated zone. *GDNF*^{*Z*/}*^{F}; Cre-Esr1l*+ mice without (C) and with (D) TM treatment. Scale bar indicates 100 µm.

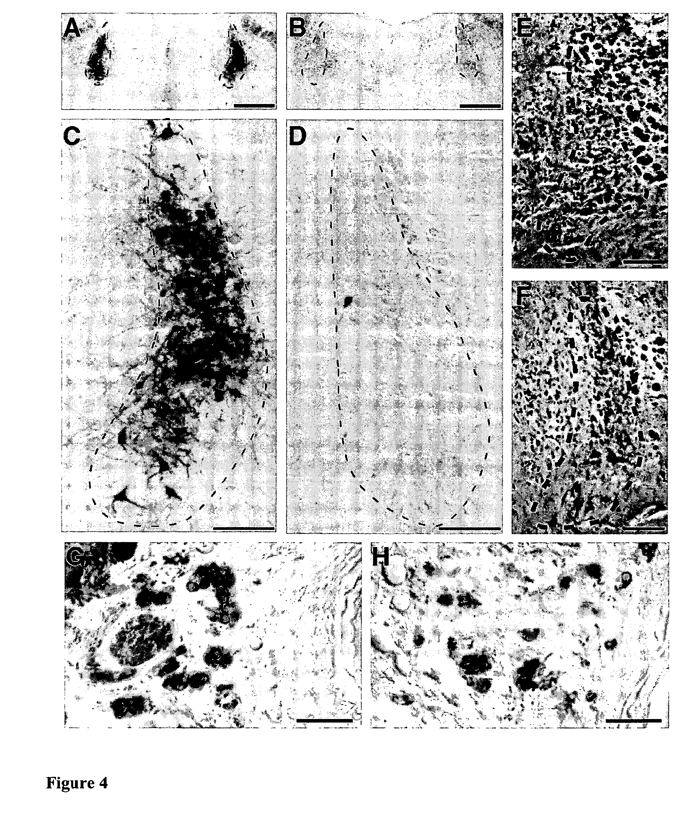

### Figure 4. LC and CB Neuronal Death After Ablation of GDNF in Adult Brain

(A-D) Hindbrain slices stained with an antibody against TH. *GDNF*^{*Z*/}*^{F}; Cre-Esr1*/+ without (A and C) and with (B and D) TM treatment. (E-F) Nissl staining in brain slices of *GDNF*^{*Z*/}*^{F}; Cre-Esr1*/+ mice without (E) and with (F) TM treatment. The stippled line highlights the locus coeruleus (LC). (G-H) Carotid body of *GDNF*^{*Z*/}*^{F}; Cre-Esr1*/+ mice without (G) and with (H) TM treatment as revealed by TH immunoreactivity. Scale bar indicates 100 µm in all panels except (A-B) panels where it represents 500 µm.

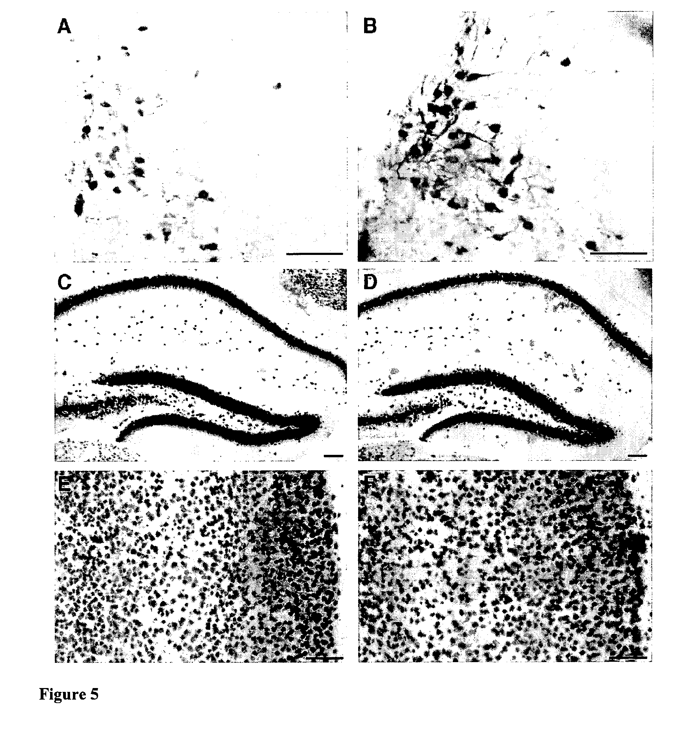

### Figure 5. Representative Brain Regions Unaffected by Adult GDNF Depletion.

(A-B) Nucleus arcuatus immunostained for TH. *GDNF*^{*Z*/}*^{F}; Cre-Esr1*/+ mice without (A) and with (B) TM treatment. (C-F) Brain slices stained with an antibody against NeuN. (C-D) hippocampus; (E-F) cortex. *GDNF*^{*Z*/}*^{F}; Cre-Esr1l*+ mice without (C and E) and with (D and F) TM treatment. Means ± S.E.M.; *, *p* < 0.05. n = 6-10 brains per group. Scale bar indicates 100 µm.

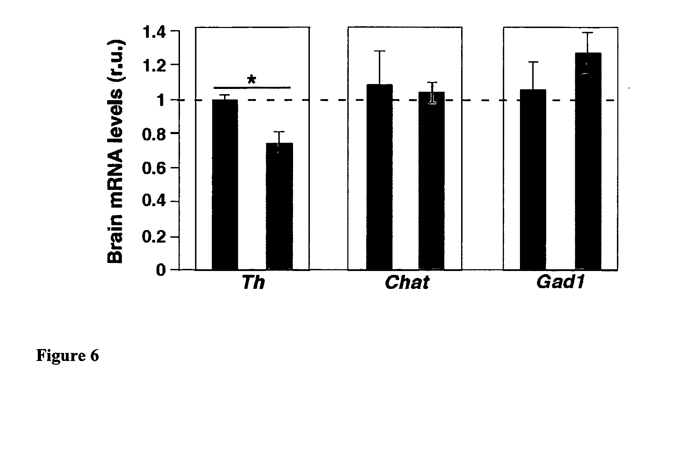

### Figure 6. Neurotransmiter mRNA levels in adult GDNF depleted animals.

Relative levels of tyrosine hydroxylase (*Th)*, choline acetyltransferase (*Chat*), and glutamate decarboxylase (*Gad1)* mRNA determined by quantitative RT-PCR. *GDNF*^{*Z*/}*^{F}; Cre-Esr1*/+ mice without (black bars) and with (gray bars) TM treatment. Means ± S.E.M.; **, p* < 0.05. *n* = 6-10 brains per group.

### Figure 7. SN after embryonic GDNF deletion

(A-B) Mesencephalic brain slices stained with an antibody against TH. *GDNF*^{+/+}*; GFAP-Cre*/*+* (A); *GDNF*^{*Z*/}*^{F}GFAP-Cre*/+ (B).

### Figure 8. GDNF Expression in Catecholaminergic Brain Targets

GDNF promoter activity as estimated by β-galactosidase enzymatic detection in *GDNF*^{*Z*/+} brains. (A-C) Coronal brain section demonstrating high GDNF expression in the striatum (B, St) and thalamus (C, Th). The region encircled by the black line (A) represents the area in thalamus with higher density of X-gal+ cells. (D-E) Coronal brain section demonstrating high GDNF expression in septum (E, Sp). The region encircled by the red line (D) represents the septal area with higher density of X-gal+ cells (F-G) GDNF expression in the subcommissural organ (SCO). Scale bar indicates 500 µm in A and D; 10 µm in B, C and E; and 50 µm in F.

### DETAILED DESCRIPTION OF THE INVENTION

The authors of the present invention have found that, surprisingly, animals carrying a conditional deletion in the gene coding for GDNF develop some of the findings of PD patients, in particular, the experiments show that (i) noradrenergic cells of the locus coeruleus, which are affected in certain neurodegenerative disorders and particularly in early stages of PD, absolutely depend on GDNF for their survival during adult life; (ii) dopaminergic neurons in the *substantia nigra* and ventral tegmental area, affected in PD, require GDNF for their survival during adult life resulting the absence of GDNF in cell death, (iii) GDNF depletion does not affect, at least during the initial stages of neurodegeneration, other CNS areas such as cortex, hippocampus or other area which show expression of tyrosine hydroxylase such as nucleus arcuatus (interestingly the nucleus arcuatus remains unaffected in PD) and (iv) depletion of GDNF in specific cells (glial cells or cells which express GFAP) during embryonic life results in the genetic compensation in other cells in the brain.

### The "targeting construct of the invention"

Thus, in a first aspect, the invention relates to a DNA construct (hereinafter referred to as "the targeting construct of the invention") which comprises at least a region of the GDNF gene flanked by recombinase a target sites wherein said region of the GDNF gene is necessary for the expression of a functional GDNF gene and the recombinase sites are arranged so that, in the presence of the recombinase, the intervening sequence is deleted.

The mouse GDNF gene structure is known from the GenEMBL record with accession number D88352. The rat GDNF gene is known from the GenEMBL record with accession number AJ011432. The human GDNF gene is known from WO9953091. It will be understood that any region of the GDNF gene can be used in the targeting construct as long as said region is required for the expression of a functional GDNF protein, either for proper transcription of the gene, for processing of the transcript, for the translation of the mRNA or for the activity of the protein. The skilled person will appreciate that the findings of Pichel et al (Nature, 1996, 382:73-76), Sanchez et al (Nature, 1996, 382:70-73) and Moore et al (Nature, 1996, 382:76-79) wherein it was found that mice harboring an homozygous deletion in the GDNF gene die 12-24 hours after birth can be used as a test for the identification of essential regions of the GDNF gene. Thus, by removing a part of the GDNF gene and testing whether the animals harboring two modified copies of the GDNF gene show the same phenotype (lethality at 12-24 h after birth) as the strains carrying a complete deletion of the gene, the region of the GDNF gene can be defined as essential. Regions of the GDNF gene which can be used in the present invention include exons, introns, the 5' or 3' untranslated regions or the regulatory region. In a preferred embodiment, the targeting construct contains at least one exon of the GDNF gene. In a still more preferred embodiment, the targeting construct contains exon 3 of the GDNF gene.

The recombinase target sites in the targeting construct of the invention can be selected from loxP sites specific for the phage P1 Cre recombinase as well as FRT sites specific for the *S*.*cereivisae* FLP recombinase. In a preferred embodiment, the recombinase target sites are loxP sites which are formed by a 34 base pair sequence comprised of two 13 bp inverted repeats sequences flanking a 8 base pair core sequence. The invention contemplates as well the use of variants of the LoxP sequence which are incapable of undergoing recombination with the wild-type loxP sequences but which are capable of high recombination efficiency with their cognate loxP variant, such as those described in WO199925851 and WO2007085906. These loxP variants are particularly useful when the genome of the target cell already contains a region flanked by wild-type loxP sequences.

The targeting construct of the invention may contain as well resistance markers which are useful for selecting those cells wherein the targeting construct has recombined with the GDNF locus. Suitable resistance markers for selecting cells which have integrated the construct in the genome include positive selection markers such as, for example, the neomycin resistance gene, conferring resistance to the aminoglycoside G418, the hygromycin phosphotransferase gene conferring resistance to hygromycin, the ODC gene conferring resistance to the ornithine decarboxylase inhibitor (2-(difluoromethyl)-DL-omithine (DFMO)), the dihydrofolate reductase gene conferring resistance to methotrexate, the puromycin-N-acetyl transferase gene conferring resistance to puromycin, the ble gene conferring resistance to zeocin, the adenosine deaminase gene conferring resistance to 9-beta-D-xylofuranosyl adenine, the cytosine deaminase gene allowing the cells to grow in the presence of N-(phosphonacetyl)-L-aspartate, thymidine kinase allowing the cells to grow in the presence of aminopterine, the Xanthine-guanine phosphoribosyltransferase gene allowing the cells to grow in the presence of xanthine and in the absence of guanine, the E.coli trpB gene allowing the cells to grow in the presence of indole instead of tryptophan, the E.coli hisD gene allowing the cells to use histidinol instead of histidine. The selection gene is usually linked to a suitable promoter for the expression of said gene in eukaryotic cells, (for example the CMV or SV40 promoters), an optimized translation initiation site (for example a site following the so-called Kozak rules or an IRES), a polyadenylation site such as for example the SV40 or phosphoglycerate kinase polyadenylation site, introns such as for example the beta-globulin gene intron.

In another aspect, the invention provides a vector which contains the targeting construct of the invention. These vectors allow the propagation of the targeting construction suitable hosts. Suitable vectors for use in the present invention include prokaryotic expression vectors such as pUC18, pUC 19, Bluescript and their derivatives mp18, mp19, pBR322, pMB9, CoIE1 , pCR1 , RP4, phages and shuttle vector such as pSA3 and pAT28, yeast expresión vectors such as 2 micro plasmids, integrative plasmids, YEP vectors, centromeric plasmids and the like, plant expression vectors such as pIBI, pEarleyGate, pAVA, pCAMBIA, pGSA, pGWB, pMDC, pMY, pORE vectors and the like, insect cell expression vectors such as pAC and pVL vectors, eukaryotic expression vectors either based in viral vectors (adenovirus, adeno-associated viruses, retroviruses and lentivirus) as well as non viral vectors such as pcDNA3, pHCMV/Zeo, pCR3.1, pEF1/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d and pTDT1.

In another aspect, the invention relates to host cells which comprise a vector or a DNA construct of the invention. Cells that can be used for the purposes of the invention are preferably eukaryotic cells, more preferably a vertebrate or invertebrate cell, insect cells or fungal cells, even more preferably the vertebrate cell is a xenopus cell, a cell isolated from a zebra fish or a mammalian cell. Preferably, the mammalian cell is a cell from a established cell line such as CHO, VERO, BHK, HeLa, COS, MDCK 293, 3T3, WI38 and the like, an embryonic stem cell, an adult stem cell or a somatic cell. In a preferred embodiment, the cell is an embryonic stem cell.

### The conditional GDNF knock-out cells of the invention

The targeting construct of the invention can be introduced into a cell and undergo homologous recombination with the GDNF gene thus resulting in the replacement of the region of the GDNF gene by the homologous region present in the targeting construct which results in the introduction of the recombinase target sites in the GDNF locus. Thus, in a further aspect, the invention relates to a host cell wherein the targeting construct of the invention has recombined with the GDNF locus. Cells wherein the recombination event has taken place are then selected for their resistance to the marker which is used together with the targeting construct.

The targeting construct or the vector containing said targeting construct is introduced in the cells being studied using any of the transfection methods known by the persons skilled in the art (see sections 9.1 to 9.5 in Ausubel, F.M. et al., Current Protocols in Molecular Biology, John Wiley & Sons Inc; ringbou edition, 2003). The cells can be transfected by means of precipitating DNA with calcium phosphate, DEAE-dextran, polybrene, electroporation, microinjection, liposome-mediated fusion, lipofection, infection by retrovirus and biolistic transfection.

Once the cells have been transfected, it is necessary to select those cells wherein the targeting construct has recombined with the homologus region in the GDNF gene. The screening process of cells containing the DNA construct of interest recombined in the genome is carried out by means of a conventional screening process (see for example Ausubel, F.M. et al., Current Protocols in Molecular Biology (1997) 9.5.1-9.5.19). To that end, the cells are transfected with a vector or mixture of vectors and, after a recovery period, they are allowed to grow in a selective medium (either a medium containing the antibiotic against which the reporter confers resistance or a minimum medium containing the antimetabolite against which the reporter confers resistance). The cell colonies growing in selective medium are isolated and allowed to grow again in selective medium. Once cells have been obtained which can grow during repeated proliferation cycles in the presence of the selection marker, it may be convenient to eliminate said marker from the cells, particularly if the cells are to be transfected with another selection marker. A cell is normally considered to stably express a marker when the expression of said marker does not decrease with successive proliferation cycles, independently of the presence of selection agent in the culture medium. It is also necessary to identify those colonies wherein the targeting construct has recombined with the GDNF locus from those wherein spurious recombination has occurred. This can be carried out by amplification of the GDNF locus using primers which allow to distinguish those cells wherein the GDNF locus contains recombinase target sites flaking the region of the GDNF gene derived from the targeting construct from those cells wherein the recombinase target sites are present somewhere else in the genome.

Cells that can be used for the purposes of the invention are preferably eukaryotic cells, more preferably a vertebrate or invertebrate cell, insect cells or fungal cells, even more preferably the vertebrate cell is a xenopus cell, a cell isolated from a zebra fish or a mammalian cell. Preferably, the mammalian cell is a cell from a established cell line such as CHO, VERO, BHK, HeLa, COS, MDCK 293, 3T3, WI38 and the like, an embryonic stem cell, an adult stem cell or a somatic cell. In a preferred embodiment, the cell is an embryonic stem cell.

### The conditional GDNF knock-out animal of the invention

The cells carrying a recombined GDNF locus wherein a region of the gene is flanked by recombinase target sites can be used for obtaining a non-human animal carrying said genetic modification. Thus, in a further aspect, the invention relates to a method for obtaining a non-human transgenic animal which comprises the step of propagating the cell of the invention and developing from said cells a viable organism, wherein said cell is non-human. In yet another aspect, the invention relates to non-human animals obtainable by the method defined above.

It will be appreciated that the method of obtaining a non-human animal can only be carried out when the cell of the invention is a stem cell which can differentiate into every cell type, including germ line cells. These are embryonic cell (EC) or embryonic germ cells (EG). Embryonic Stem cells (ES) derive from the inner cell mass of the preimplantation blastocyst stage embryo. Several types of mouse and human embryonic stem cells are known and established, like for example the mouse ES TBV2, R1, D3 cells. These cells can be cultured in vitro under appropriate culture conditions in an undifferentiated state and retain the ability to resume normal in vivo development as a result of being combined with blastocyst and introduced into the uterus of a pseudopregnant foster mother. Embryonic germ cells (EG) derive from cultured Primordial Germ Cells of the foetal genital ridge (PGCs). Typically, the ES and EG cells of the invention carrying a modified GDNF locus are injected into a host blastocyst, i.e., the blastocoel of the host blastocyst, or co-cultured with eight-cell to morula-stage ova, i.e., zona-free morula, so that modified ES cells are preferentially incorporated into the inner cell mass of the developing embryo. With blastocyst injection transgenic offspring are termed chimeric, as some of their cells are derived from the host blastocyst and some derive from the modified ES cells. The host embryos are transferred into intermediate hosts or surrogate pseudopregnant females for continuous development. The process will result in chimeric animals whose somatic and germ line tissue comprise a mixture of cells derived from the genetically-engineered ES cells and the recipient blastocyst. Typically, the stem cells and the blastocysts are obtained from animals having different skin pigmentations so that the chimeric animals will carry patches of different colours. Thus, these chimeric animals are then back-crossed with their siblings until a germ-line transgenic animal is obtained, i.e. an animal wherein the transgene or the modified GDNF locus is present in the germ cells of the animal so that the trait can be passed to the offspring of the animal through reproduction. Germ-line transgenic animals can be identified, for example, by observing offspring for the presence of the trait or by examining the germ cells of the transgenic animal for the presence of a transgene, incorporated in a manner consistent with heritability. These animals are then crossed to other animals so as to obtain animals monozygotic for the desired trait but which do not longer show mosaicism. The monozygotic animals are then crossed among themselves so as to obtain dizygotic animals wherein both copies of the GDNF locus are modified.

It is to be understood that the transgenic non-human mammals described herein can be produced by methods other than the ES cell method described above, for example by the pronuclear injection of targeting construct into the pronuclei of one-cell embryos or other gene targeting methods which do not rely on the use of a transfected ES cell.

Any suitable non-human mammal can be used to produce the transgenic non-human mammal described herein. For example, a suitable mammal can be a rodent (e.g., mouse, rat), a rabbit, a pig, a sheep, a goat or a cow provided that the specific line(s) of the animal are selected for general good health, good embryo yields, good pronuclear visibility in the embryo, and good reproductive fitness In a preferred embodiment, the animal is a mouse. Strains such as C57BL/6 or C57BL/6 x DBA/2 Fit, or FVB lines are often used (obtained commercially from Charles River Labs, Boston, Mass., The Jackson Laboratory, Bar Harbor, ME, or Taconic Labs.). Preferred strains are the 129sv strain as well as those with strains having H 2b, H-26 or H-2q haplotypes such as C57BL/6 or DBA/1.

The transgenic animal of the invention can also be used for obtaining animals wherein both loci of GDNF are modified. In a preferred embodiment, the animals carrying two modified GDNF loci are obtained by breeding animals with one modified GDNF locum and selecting from the offspring those animals wherein both GDNF loci are modified. Thus, in another aspect, the invention relates to a method for the generation of transgenic non-human animals which comprises the step of breeding animals wherein one copy of the GDNF locus is modified by recombinase target sites and selecting those animals wherein both GDNF loci contain the recombinase target sites. In another aspect, the invention relates to transgenic animals obtainable by the above method and wherein both copies of the GDNF gene are modified so as to contain recombinase target sites flanking an essential region of the GDNF gene.

The cells and animal of the invention carrying a modified GDNF locus can also be used to obtain a cell carrying an inactive GDNF gene by contacting said cell with a compound which is capable of inducing the expression of a recombinase specific for the target sites present in the modified GDNF locus, thus resulting in the excision of the region of the GDNF region flanked by the recombinase target sites. Thus, in another aspect, the invention relates to a method for obtaining a cell or a non-human animal carrying an inactive GDNF gene comprising the steps of
(a) contacting a cell of the invention or a non-human transgenic animal of the invention with a compound which is capable of inducing the expression of a recombinase specific for the target sites present in the GDNF and
(b) selecting those cells or animals wherein the recombinase has catalyzed the excision of the functional part of the GDNF locus.

A compound capable of inducing the expression of a recombinase specific for the target sites present in the GDNF, as used herein, refers to any molecule which is capable of inducing the expression of a recombinase in a cell. Preferably, this molecule is a polypeptide with recombinase activity or a nucleic acid which codes for a polypeptide with such activity. The inducer of recombinase activity must be brought inside the cell. If the molecule is a nucleic acid, it can be introduced into the cell by means of any transfection method known in the art as described previously. If the molecule is a protein, the protein must be modified with a peptide which can promote the translocation of the protein to the cell interior, such as the Tat peptide derived from the HIV-1 TAT protein, the third helix of the Antennapedia homeodomain protein from *D*.*melanogaster*, the VP22 protein of the herpes simplex virus and arginine oligomers (Lindgren, A. et al., 2000, Trends Pharmacol. Sci, 21:99-103, Schwarze, S.R. et al., 2000, Trends Pharmacol. Sci., 21:45-48, Lundberg, M et al., 2003, Mol. Therapy 8:143-150 and Snyder, E.L. and Dowdy, S.F., 2004, Pharm. Res. 21:389-393).

Recombinases contemplated for use in the practice of the present invention include Cre recombinase, FLP recombinase, the R gene product of Zygosaccharomyces (Onouchi et al. (1995) Mol Gen Genet 247:653-660), and the like. In a preferred embodiment, the recombinase is a Cre recombinase which include the Cre recombinase of the P1 phage as described originally by Abreinski and Hoess (J. Biol. Chem. 1984, 259:1509- 1514) as well as variants thereof having broadened specificity such as those described in WO2000060091.

In a preferred embodiment, the compound which induces the expression of the recombinase is a nucleic acid coding for a recombinase and the nucleic acid is brought into the cell by the use of a viral vector, preferably, an adenoviral vector. Any adenoviral known in the art can be used for the purposes of the present invention such as Ad12 (subgenus A), Ad3 and Ad7 (subgenus B), Ad2 and Ad5 (subgenus C), Ad8 (subgenus D), Ad4 (subgenus E), Ad40 (subgenus F), and other known non-human adenoviruses that may originate from species such as swine, sheep, bovine cattle and birds.

In another aspect, the invention relates to non-human animals and cells obtainable by the method as described above.

The targeting constructs of the invention can also be used together with a construct coding for a recombinase whose expression and/or activity can be regulated by an external signal so as to obtain cells carrying a conditional GDNF knock-out cell that can be used to obtain cells carrying a deletion in the GDNF locus by contacting the cell with a compound that activates the expression and/or activity of the recombinase. Thus, in another aspect, the invention relates to a method for the generation of a conditional knock-out cell in the GDNF gene comprising the steps of:
(a) introducing a nucleic acid molecule comprising the targeting construct of the invention into a cell which expresses a recombinase whose expression and/or activity can be regulated by an external signal and
(b) selecting cells wherein the DNA construct introduced in step (a) has recombined with the GDNF locus.

In another aspect, the invention relates to a method for the generation of a conditional knock-out cell in the GDNF gene, comprising the steps of:
(a) introducing into a cell a first nucleic acid molecule comprising the targeting construct of the invention,
(b) selecting those cells wherein the DNA construct has recombined with the GDNF locus,
(c) introducing into the cells selected in step (b) a second nucleic acid molecule coding for a recombinase whose expression and/or activity can be regulated by an external signal and
(d) selecting cells which have integrated into their genome the second nucleic acid molecule.

In yet another aspect, the invention relates to a method for the generation of a conditional knock-out cell in the GDNF gene, comprising the steps of:
(a) introducing into a cell a first nucleic acid molecule comprising the targeting construct of the invention and a second nucleic acid molecule coding for a recombinase whose expression and/or activity can be regulated by an external signal and
(b) selecting those cells wherein the DNA construct of the first nucleic acid molecule has recombined with the GDNF locus and which have incorporated into their genome said second nucleic acid molecule.

The "recombinase whose activity and/or expression" can be activated by an external signal, as used herein, refers to a recombinase which is either not expressed or is expressed in a non-functional form or is expressed in a functional form but does not reach the nucleus of the cell and which, in the presence of an external signal, is either expressed, activated or translocated into the nucleus.

In one embodiment, the nucleic acid coding for the recombinase is operably linked to a regulatable promoter which has low levels of background expression and which is only expressed upon the addition of a compound which activates transcription mediated by such promoter. Examples of well known inducible promoters are: an androgen or estrogen promoter, a metallothionein promoter, or a promoter responding to ecdysone. In a preferred embodiment, the inducible promoter is an interferon inducible promoter which is known to be capable of promoting interferon-dependent expression of the Cre recombinase (Kuehn et al., 1995, Science, 269:1427-1429).

In another embodiment, the recombinase is constitutively expressed as a fusion protein with an activable NLS. An "activable NLS", as used herein, refers to a nuclear localization signal which is capable of targeting any passenger protein to the nucleus only in the presence of a external signal. In a preferred embodiment, the NLS derives from a nuclear receptor. Nuclear receptors suitable for use in the present invention include PPARs (receptors activated by peroxisome proliferators) which are translocated in the presence of 15-desoxy-[Delta]-prostaglandina J2, retinoic acid receptors (RX) which are transloicated into the nucleis un the presence of the alpha. beta or gamma isomers of teh 9-cis-retinoic cid, farnesoid X receptors (FXRs), activables by retinouc acid and TTNPB, liver X receptors (LXR) activables by 24-hydroxui-cholesterol, benzoate X receptors, activables by 4-amino-butil benzoate, androstane constitutive receptor, activable by androstanol, pregnane receptors, inducible by pregnolone-16 carbonitrilo and steroid receptors and xenobiotics, indicible by rifmapycin, progesterone receptors activable by medroxiprogesterone and agonists and antagonists of mifepristona and 19-nortestorene derivatives, glucocorticoides receptors actibvable by glucocorticoides, thyroid hormone receptors activable by T3 and/or T4 and estrogen receptors activable by estrogen and derivatives as 17-betaestradiol and estradiol.

Preferrably, the NLS which is activable corresponds to the ligand binding domain (LBD) of the estrogen receptor essentially as it has been described in WO0228175. As "ligand binding domain or LBD", as used herein, it is understood the sequence of the ligand binding domain of a estrogen receptor and variants thereof comprising one or more mutations resulting from insertion, deletion and/or substitutions of one or more residues and which respond in a differential manner to the natural ligand of the receptor (estradiol, estriol or estrone) and to the different agonists and antagonists of the receptor such as ICI 164384, RU 54876, dietilestilbestrol, raloxifeno, zuclomifeno y toremifeno.

The recombinase and the NLS can be arranged in any orientation with respect to each other. Thus, the invention comprises constructs wherein the C-terminal region of the LBD is connected to the N-terminal region of the recombinase and constructs wherein the C-terminal region of the recombinase is connected to the N-terminal region of the LBD. Additionally, the invention contemplates the use of LBD variants which show an affinity towards different estrogen receptors which is different from that of the natural LBD. Thus, the invention contemplates the use of LBD carrying a G512R mutation (the numbering relative toe the human estrogen receptor). Said mutation results in an affinity of the LBD towards the natural ligand which 1000-fold lower than that of the wild-type receptor but which does not affect the affinity of the LBD towards 4-hydroxytamoxifen. In this way, it is possible to promote nuclear translocation of the recombinasa-BLS fusion protein by using 4-hydroxytamoxifen without affecting the estrogen response in the animal. In another embodiment, the estrogen receptor LBD contains the mutations G400V/M543A/L544A, which results in a polypeptide which can be activated using lower doses of 4-hidroxitamoxifen and by tamoxifen than those required to activate the G512R mutant. Alternatively, the nuclear receptor LBD contains the M543 and L544A mutations, thus resulting in a molecule which shows a 4-hidroxitamoxifen and tamoxifen sensitivity which is 10-fold higher than that shown by the G400V/M543A/L544A mutant.

In a preferred embodiment, the activable NLS is the ligand binding domain of the estrogen receptor or the G521R variant thereof as described in Feil et al. (Proc.Natl.Acad.Sic. USA, 1996, 93:10887-10890).

The nucleic acid coding for the recombinase fused in frame to an NLS is usually operably linked to a promoter so as to obtain sufficient intracellular levels of the recombinase. Exemplary constitutive promoters contemplated for use in the practice of the present invention include the CMV promoter, the SV40 promoter, the DHFR promoter, the mouse mammary tumor virus (MMTV) steroid-inducible promoter, Moloney murine leukemia virus (MMLV) promoter, elongation 15 factor la (EF1a) promoter, albumin promoter, APO A1 promoter, cyclic AMP dependent kinase I1 (CaMKII) promoter, keratin promoter, CD3 promoter, immunoglobulin light or heavy chain promoters, neurofilament promoter, neuron specific enolase promoter, L7 promoter, CD2 promoter, myosin light chain kinase promoter, HOX gene promoter, thymidine kinase (TK) promoter, RNA Pol I1 promoter, MYOD promoter, MYF5 promoter, phophoglycerokinase (PGK) promoter, Stf 1 promoter, Low Density Lipoprotein (LDL) promoter, chicken 6-actin promoter (used in conjunction with ecdysone response element) and the like.

In those embodiments wherein the cell is simultaneously or sequentially transfected with a first nucleic acid coding for a recombinase whose expression and/or activity can be regulated by an external signal and a second nucleic acid coding for the targeting construct, it is usually convenient that both nucleic acids are provided in different vectors, each of them containing a different selection marker. It will be appreciated that any combination of the markers described above can be used for the purposes of the present invention.

Recombinases suitable for the present invention are those mentioned above.
Preferably, the recombinase is the Cre recombinase.

In another aspect, the invention relates to a cell which has been generated by the method as defined above. Cells that can be used for the generation of conditional GDNF knock out are those mentioned above. Preferably, the cell is an embryonic stem cell.

The conditional GDNF knock out cells of the invention can be contacted with the compound which is capable of inducing the expression and/or the activity of the recombinase so as to obtain a cell carrying a deletion in at least one of the GDNF alleles. Thus, in another aspect, the invention relates to a method of obtaining a cell carrying an inactive GDNF gene comprising the steps of
(a) contacting a conditional GDNF knock-out cell of the invention with a compound which is capable of inducing the expression and/or activity of the recombinase and
(b) selecting those cells wherein the recombinase has catalysed the excision of a functional part of the GDNF locus.

It will be appreciated that the compound required for the activation of the recombinase will depend on the type of inducible promoter operably linked to the recombinase coding sequence or on the type of activable NLS which is fused to the recombinase. If an interferon-inducible promoter is used, then the cells can be contacted with an interferon molecule, preferably interferon of the type IFN-α, IFN-β, IFN-δ, IFN-ε, IFN-κ, IFN-τ e IFN-ω. If the recombinase is synthesised as a fusion protein with an activable NLS, then the compound that needs to be added is an activator of the NLS. In a preferred embodiment, the activable NLS is the ligand binding domain of the estrogen receptor and the activator of the NLS is a compound selected from an estrogen, such as estradiol or an anti-estrogen such as tamoxifen or 4-hydroxytamoxifen.

The cells carrying a conditional knock out mutation in the GDNF gene can be propagated to obtain a transgenic animal harboring said modified GDNF locus. Thus, in another aspect, the invention relates to a method for the generation of transgenic non-human animal, comprising the step of propagating the cell carrying a modified GDNF locus and developing from said cell a viable organism, wherein said cell is non-human.

In another aspect, the invention relates to methods for the generation of a transgenic non-human animal, comprising the steps of
(a) introducing into a cell a nucleic acid molecule comprising the targeting construct of the invention wherein said cell is a embryonic stem cell or an non-human adult stem cell,
(b) selecting those cells wherein the DNA construct has recombined with the GDNF locus,
(c) developing from said cell a viable non-human animal,
(d) breeding the non-human animals obtained in step (c) with a strain from the same non-human species which contains into their genome a second nucleic DNA construct coding for a recombinase whose expression and/or activity can be regulated by an external signal.
(e) selecting those animals from the offspring which contain both the first nucleic acid molecule and the second nucleic acid molecule.

In another aspect, the invention relates to a method for the generation of a transgenic non-human animal, comprising the steps of
(a) introducing into a cell a first nucleic acid molecule comprising the targeting construct of the invention,
(b) selecting those cells wherein the DNA construct has recombined with the GDNF locus,
(c) introducing into the cells selected in step (b) a second nucleic acid molecule comprising the DNA construct of a second nucleic acid coding for a recombinase whose expression and/or activity can be regulated by an external signal,
(d) selecting cells which have integrated the second nucleic acid molecule and
(e) developing from said cell a viable non-human animal,

It will be appreciated that the methods for obtaining transgenic animals carrying a conditional deletion in the GDNF gene are carried out essentially as describe before for the methods for obtaining animals harboring a modified GDNF gene. In a preferred embodiment, the animal is a non-human mammal. In another embodiment, the recombinase whose expression and/or activity can be regulated by an external signal is a recombinase which is operably linked to an inducible promoter. In another aspect, recombinase whose expression and/or activity can be regulated by an external signal is a recombinase coupled to the ligand binding domain of the estrogen receptor. In a yet more preferred embodiment, the recombinase is the Cre recombinase.

In another aspect, the invention relates to a transgenic non-human animal produced by any of the above mentioned methods. The animal can either carry a single modified GDNF allele. However, these animals may still produce sufficient amount of GDNF after inactivation of said single modified allele so that they may not develop signs of PD. Thus, in another aspect, the invention relates to a method for the generation of a transgenic animal wherein both copies of the GDNF loci are modified by the insertion of recombinase target sites flanking an essential region of the GDNF locus and which contain a recombinase whose expression and/or activity is regulated by an external signal. Preferably, animals carrying a single modified GDNF locus are then crossed so as to obtain animals wherein both GDNF alleles are modified so that activation of the recombinase results in a loss of function of both genes.

The transgenic animals of the invention which contain the modified GDNF locus with recombinase target sites and the transgene coding for a recombinase whose expression and/or activity can be induced by an external signal can be contacted with said signal, thus leading to an increase in the activity or expression of the recombinase so a recombination event between the recombinase target sites takes place, thus resulting in the excision of an essential part of the GDNF gene. Thus, in another aspect, the invention relates to a method for obtaining a non-human animal carrying an inactive GDNF gene which comprises contacting an animal of the invention with a compound which is able to induce the expression or activity of the recombinase so that the recombinase-mediated excision of a functional part of the GDNF locus takes place. The invention contemplates the use of a recombinase whose activity is regulated at the expression level by the use of a inducible promoter upstream of the nucleic acid coding for the recombinase. In such case, induction of the recombinase requires the addition of a compound which triggers expression of genes downstream of the inducible promoter. However, preferably, the transgenic animals contain a recombinase coupled to an activable NLS so that the activation of the recombinase is achieved by adding a compound which activates the NLS, i.e. it allows the NLS to target any attached protein to the nucleus. In a preferred embodiment, the NLS is the ligand binding domain of the estrogen receptor. More preferably, the activation of the NLS is achieved using tamoxifen.

It will be appreciated that the determination of whether the compound is capable of inducing the expression/activity of the recombinase can be determined using standard methods known to the skilled person. For instance, a sample of a tissue of the organism can be isolated and a region of the GDNF gene can be amplified so as to detect any deletion in the gene.

The skilled person will appreciate that the compound capable of inducing the activity and/or expression of the recombinase must be administered in adequate amounts so that the intracellular concentration of recombinase is sufficient for excising the region of the GDNF gene. The administration can be carried out by any means known to the skilled person but will depend on the nature of the compound being administered. For instance, the compound can be administered intravenously, intraperitoneally, subcutaneously, intramuscularly, topically, intradermally, intranasally or intrabronchially. Preferrably, the compound is administered intraperitoneally. It will also be appreciated that the compound must be administered only to adult organisms because the administration in an early developmental stage may result in lethality, as observed in homozygous GDNF knock out mice (Pichel et al., *supra*.). Preferably, if the animals are mice, administration of the compound is carried out when they are at least two months old.

In another aspect, the invention relates to non-human transgenic animals obtained by the method described above. In another aspect, the invention relates to an organ, tissue or cell isolated from a non-embryonic non-human transgenic animal of the invention. Organs which can be preferably isolated from the animals of the present invention include organs of any region of the body, including head and neck (face, orbit, eye, mouth, tongue, teeth, nose, ears, scalp, larynx, pharynx, salivary glands, meninges, brain, thyroid and parathyroid gland), back and spine (vertebra and spinal cord), thorax (mammary gland, ribs, lungs, heart, mediastinum, esophagus and diaphragm), abdomen (peritoneum, stomach, duodenum, intestine, colon, liver, kidney, adrenal gland, appendix and pancreas), pelvis (sacrum, coccyx, ovaries, fallopian tube, uterus, vagina, vulva, clitoris, perineum, urinary bladder, testicles, rectum and penis) and limbs (muscle, bone, nerves, hand, wrist, elbow, shoulder, hip, knee or ankle). In a preferred embodiment, the organ which is isolated is brain.

The animals carrying a deletion in the GDNF gene undergo a series of events that resemble certain neurodegenerative diseases. The defects appear in the animals of the invention include (i) a decrease in the number of some dopaminergic and noradrenergic nuclei in the brain, in particular, the substantia nigra and the tegmental ventral area, (ii) a disappearance of the locus coeruleus and (iii) a decrease in the amount of fibres in the dorsal and ventral striated nuclei. Thus, in another aspect, the invention relates to a method for the identification of compounds suitable for the treatment of a neurodegenerative disease comprising
(a) contacting a non-human animal carrying a deletion in the GDNF gene with a test compound and
(b) selecting those compounds which prevent or ameliorate neuronal degeneration

The compound to be assayed is preferably not isolated but forms part of a more or less complex mixture derived from a natural source or forming part of a library of compounds. Examples of libraries of compounds which can be assayed according to the method of the present invention include, but are not limited to, libraries of peptides including both peptides and peptide analogs comprising D-amino acids or peptides comprising non-peptide bonds, libraries of nucleic acids including nucleic acids with phosphothioate type non-phosphodiester bonds or peptide nucleic acids, libraries of antibodies, of carbohydrates, of compounds with a low molecular weight, preferably organic molecules, of peptide mimetics and the like. In the event that a library of organic compounds with a low molecular weight is used, the library can have been preselected so that it contains compounds which can be easily delivered to the vicinity of the areas undergoing degeneration. The compounds can thus be selected based on certain parameters such as size, lipophilicity, hydrophilicity or capacity to form hydrogen bonds. The compounds to be assayed can alternatively form part of an extract obtained from a natural source. The natural source can be an animal, plant source obtained from any environment, including but not limited to extracts of land, air, marine organisms and the like.

In a second step, the method for the identification of the compounds suitable for the treatment of neurodegenerative diseases includes determining the degree of neurodegeneration in the presence of the test compound in comparison with the vehicle. Neurodegeneration can be assessed by immunohistochemical analysis of selected brain areas using tyrosine hydroxylase as marker in dopaminergic nuclei (substantia nigra, ventral tegmental area and locus coeruleus) as described in example 1 or detection of total number of cells using the Nissl staining as well as by determining dopamine and metabolite levels. Alternatively, it is possible to measure tests of cognitive ability as well as sensory and motor alterations such as the test of locomotor activity/open field behaviour, the sensorimotor battery composed of a series of tests to evaluate balance, strength, coordination as well as initiation and speed of movement (Wozniak et al., 2004, Neurobiol.Dis. 17:403-414), the rotarod to measure continuous motor coordination and balance

In a further aspect, the invention relates to the use of a non-human animal carrying a conditional deletion in the GDNF gene for the study of the mechanisms involved in neuronal degeneration induced by GDNF deficiency. The animals can be used to study the onset and progression of the disease by contacting the animals with the agent which causes activation of the recombinase. In those animals wherein the recombinase is activated by estrogen-induced translocation to the nucleus, activation of the recombinase and subsequent inactivation of the GDNF genes takes place upon administration of tamoxifen or a variant thereof showing reduced activity on endogenous estrogen receptors. Once the deletion of the GDNF genes has occurred, the lack of GDNF leads to the progressive reduction of TH+ cells and in the total number of neurons in the *substantia nigra* and in the in the ventral tegmental area.

In another aspect, the invention relates to the use of a non-human animal carrying a conditional deletion in the GDNF gene for the identification of GDNF agonists and activators. As used herein, "a GDNF agonist" refers to any compound capable of signaling through GDNF receptors. As used herein, "a GDNF activator" refers to any compound which results in increased levels of GDNF by transcriptional and/or translational activation of the GDNF gene. According to this aspect of the invention, the animals are treated with the agent that induces activation of the recombinase, thus leading to the inactivation of the GDNF gene. Subsequently, the animals are treated with an agent or collection of agents whose activity as GDNF agonist or activator wants to be tested. It will be understood that the method applied to animals wherein both copies of the GDNF gene has been inactivated will only allow the identification of GDNF agonists, since no transcriptional and translation activation of the GDNF gene from the disrupted GDNF gene is possible. On the other hand, when the method is applied to animals wherein only one copy of the GDNF gene is inactivated will allow the identification of GDNF agonists and activators, since it will not be possible to exclude whether the GDNF-like effect is caused by direct stimulation of the GDNF receptor by the test compound or by an increase in the levels of GDNF produced from the non-disrupted GDNF gene. Those compounds which prevent the degeneration of dopaminergic nuclei in the brain of the GDNF knock out mice are then selected. According to the invention, "putting in contact" an animal with the candidate compound includes any possible way of taking the compound to the central nervous system wherein degeneration of dopaminergic neurons takes place. Thus, in the event that the candidate compound is a molecule with low molecular weight with sufficient hydrophobicity, it is enough to administer said molecule intravenously. In the event that the candidate compound is a molecule with a high molecular weight (for example, biological polymers such as a nucleic acid or a protein), it is necessary to provide the means so that this molecule can access the central nervous system. In the event that the candidate molecule is a nucleic acid, conventional gene therapy methods aimed at therapeutic delivery of genes into the brain. In the event that the candidate compound is a protein, the protein can be administered by stereotaxic administration in the vicinity of the dopaminergic nuclei which undergo degradation in the absence of GDNF.

The compound to be assayed is preferably not isolated but forms part of a more or less complex mixture derived from a natural source or forming part of a library of compounds. Examples of libraries of compounds which can be assayed according to the method of the present invention include, but are not limited to, libraries of peptides including both peptides and peptide analogs comprising D-amino acids or peptides comprising non-peptide bonds, libraries of nucleic acids including nucleic acids with phosphothioate type non-phosphodiester bonds or peptide nucleic acids, libraries of antibodies, of carbohydrates, of compounds with a low molecular weight, preferably organic molecules, of peptide mimetics and the like. In the event that a library of organic compounds with a low molecular weight is used, the library can have been preselected so that it contains compounds which can access the cell interior more easily. The compounds can thus be selected based on certain parameters such as size, lipophilicity, hydrophilicity, capacity to form hydrogen bonds.

The compounds to be assayed can alternatively form part of an extract obtained from a natural source. The natural source can be an animal, plant source obtained from any environment, including but not limited to extracts of land, air, marine organisms and the like.

In another aspect, the invention relates to the use of a non-human animal carrying a conditional deletion in the GDNF gene for the optimization of the delivery into the animal of GDNF or agonists thereof. In this use, animals wherein a deletion of GDNF has been induced by tamoxifen-induced recombinase activity are then treated with GDNF or a variant thereof which is applied to the animal using different administration routes, dosage regimes or pharmaceutical formulations. Administration routes suitable for allowing access of GDNF to the central nervous system include topical in the form, e.g., of creams, solutions, suspensions, heptafluoroalkane aerosols and dry powder formulations, oral administration in the form of tablets, capsules, syrups, powders or granules; intravenous, intraperitoneal, subcutaneous, intramuscular, rectal, intradermal, intranasal or intrabronchially. The GDNF compositions for testing in the present invention may be obtained by conventional procedures using conventional pharmaceutical excipients, well known in the art. Thus, compositions intended for oral use may contain, for example, one or more colouring, sweetening, flavouring and/or preservative agents. Suitable pharmaceutically acceptable excipients for a tablet formulation include, for example, inert diluents such as lactose, sodium carbonate, calcium phosphate or calcium carbonate, granulating and disintegrating agents such as corn starch or algenic acid; binding agents such as starch; lubricating agents such as magnesium stearate, stearic acid or talc; preservative agents such as ethyl or propyl p-hydroxybenzoate and anti oxidants such as ascorbic acid. Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Dosage regimes suitable for testing GDNF activity include any suitable monodosage or multidosage regime and any suitable compound weight to body weight ratio like, for example, in the range of 0.0035 µg to 20 µg/kg body weight/day or 0.010 µg to 140 µg/kg body weight/week. Desirably a therapeutically effective amount is in the range of 0.025 µg to 10 µg/kg, for example, at least 0.025, 0.035, 0.05, 0.075, 0.1, 0.25, 0.5, 1.0, 1.5, 2.0, 2.5, 3.0, 3.5, 4.0, 5.0, 6.0, 7.0, 8.0, or 9.0 µg/kg body weight administered daily, every other day, or twice a week. In addition, a therapeutically effective amount can be in the range of 0.05 µg to 20 µg/kg, for example, at least 0.05, 0.7, 0.15, 0.2, 1.0, 2.0, 3.0, 4.0, 5.0, 6.0, 7.0, 8.0, 10.0, 12.0, 14.0, 16.0, or 18.0 µg/kg body weight administered weekly, every other week, or once a month. Dosage regimes may be adjusted to provide the optimum therapeutic response. For example, several divided doses may be administered daily, every other day, twice weekly, weekly, or every other week. Alternatively, the dosage may be administered continuously for a given time period, such as minutes, hours or days.

Those administration routes, dosage regimes and pharmaceutical formulations which are more efficient in preventing neuronal degeneration upon GDNF depletion are then selected for possible application in the clinical assays for the treatment of patients suffering from PD.

### The tissue-specific GDNF knock-out animal models of the invention

The authors of the present invention have also attempted to provide an animal wherein the disruption of the GDNF gene takes place in the embryonic life so as to achieve a complete GDNF depletion. For this purpose, the authors have created a transgenic animal carrying a first transgene wherein one essential part of the GDNF region is flanked by recombinase target sites and a second transgene comprising the coding sequence of a recombinase under the control of a tissue-specific promoter which is expressed during early embryonic development. However, the authors observed that animals carrying both transgenes did no show any changes in TH+ neuron counts either in *GDNF* heterozygous mice or in the embryonic *GDNF* knockouts restricted to GFAP-producing cells. Surprisingly, the early embryonic GDNF knock out heterozygotes it was observed an increase of brain *GDNF* mRNA content, suggesting that the lack of GDNF in glial cells (and in neurons derived from radial glia) was compensated by over-production of GNDF in other cell types.

Thus, in a further aspect, the invention relates to a method for the generation of a conditional knock-out cell in the GDNF locus comprising the steps of
(a) introducing the targeting construct of the invention into a cell which contains within its genome a gene coding for a recombinasa operably linked to a tissue-specific promoter and
(b) selecting those cells wherein the targeting construct has recombined with the GDNF locus.

Accordingly, in a further aspect, the invention relates to a method for the generation of a conditional knock-out cell in the GDNF gene comprising the steps of
(a) introducing the targeting construct of the invention into a cell
(b) selecting cells wherein the DNA construct has recombined with the GDNF locus.
(c) introducing into the cells selected in step (b) a second nucleic acid molecule comprising a gene coding for a recombinase which is operably linked to a tissue specific promoter and
(d) selecting cells which have integrated into their genome the second nucleic acid molecule.

In another aspect, the invention relates to a method for the generation of a conditional knock-out cell in the GDNF gene, comprising the steps of:
(a) introducing into a cell a first nucleic acid molecule comprising the targeting construct of the invention and a second nucleic acid molecule coding for a recombinase which is operably linked to a tissue-specific promoter and
(b) selecting those cells wherein the DNA construct has recombined with the GDNF locus and which have incorporated into their genome said second nucleic acid molecules.

In a preferred embodiment, the first and the second nucleic acid molecule carry a selection marker which is different between both nucleic acid molecules. Any combination of the selection markers described above will be suitable for carrying out the present invention.

Tissue-specific promoters that can be used in the second nucleic acid molecule in the context of the present invention include any promoter which is capable of triggering transcription of the downstream gene in a limited set of tissues. Non-limiting examples of said promoters include
- a stomach-specific promoter such as the H/K-ATPase beta subunit promoter, the K19 promoter, the metallothionein promoter, the TFF1 promoter, the TFF2 promoter, the FOXa/HNF2 gamma promoter
- a pancreas-specific promoter such as the elastase promoter, the Pdx-1 promoter, the insulin promoter or the phosphoglycerate kinase promoter,
- a lung-speficic promoter such as the Clara cell secretory protein promoter, the surfactant protein C promoter,
- a breast-specific promoter such as the mouse mammary tumor virus promoter or the whey acidic protein promoter,
- a skin-specific promoter including the keratin promoter or the K14 promoter,
- an esophagus-specific promoter such as the EBV L2 promoter,
- a liver-specific promoter such as the major urinary protein promoter or the albumin promoter,
- a colon-specific promoter such as the villin promoter of FABP-TS4 promoter
- a prostate-specific promoter such as the cryptidin-2 promoter, the prostate-specific antigen promoter, the C(3)1 promoter the prostate secretory protein of 94 amino acids (PSP94) promoter or the probasin promoter.
- A kidney-specific promoter such as the uromodulin promoter, the Tamm-Horsfall protein promoter or the type 1 gamma-glutamyl transpeptidase promoter,
- a bladder-specific promoter such as the uroplakin promoter or urohingin promoter,
- an uterus-specific promoter such as the uteroglobin promoter.

Additionally, any tissue-specific promoter described in the TiProD tissue-specific promoter database as described by Chen,X. et al., (Nucleic Acids Res., 2006, 34, Database Issue) can be used in the context of the present invention. In a preferred embodiment, the tissue-specific promoter is a neural cell specific promoter. Exemplary neural cell specific promoters include the promoters listed in the Lockery Lab Neuron-Specific Promoters Database, (available in "chinook.uoregon.edulpromoters.html") as well as the a c-kit promoter, specific for CA1, CA2 and CA3 regions of the hippocampus, the anterior region of the dentate gyrus, and to the ganglion cell layer of the retina; the calcium/calmodulin kinase 11 alpha promoter that drive the expression of high levels in hippocampus, cortex, and amygdala, and lower levels were detected in striatum, thalamus, and hypothalamus; the NMDA-type glutamate receptor GluRepsilon3 subunit gene that drive the expression to cerebellar granule cell. In a preferred embodiment, the neural-specific promoter is the glial fibrillary acidic protein (GFAP) promoter.

The recombinase that is encoded by the second polynucleotide is a recombinase as previously defined, including the FLP recombinase, the Cre recombinase and the the R gene product of *Zygosaccharomyces*.

In another aspect, the invention provides a cell which has been generated by a method as defined previously and which has been selected for the presence of a first transgene wherein one essential part of the GDNF region is flanked by recombinase target sites and a second transgene comprising the coding sequence of a recombinase under the control of a tissue-specific promoter. Depending on the type of promoter used, the recombinase will not be expressed, in which case the cell will preserve the two genes. On the other hand, if the particular tissue-specific promoter allows expression of the recombinase, then the transgene containing the floxed GDNF allele will undergo removal of the essential part resulting in a cell with a homo- or heterozygous deletion in the GDNF gene.

Practically, any cell known in the art can be used for the purposes of the present invention, including those cell types defined above such as eukaryotic cells, in particular, Xenopus cells, zebra fish cells or mammalian cells, more preferably embryonic stem cell, adult stem cell or somatic cells.

In a another aspect, the invention provides a method for the generation of a transgenic non-human animal which comprises the step of propagating the embryonic stem cell carrying the two transgenes and developing from said cell a viable non-human organism.

In another aspect, the invention provides a method for the generation of a transgenic non-human animal comprising the steps of
(a) introducing into a cell a first nucleic acid molecule comprising the DNA construct comprising at least a region of the GDNF gene flanked by recombinase target sites, wherein said region of the GDNF gene is necessary for the expression of a functional GDNF protein and wherein said recombinase target sites are oriented so that the intervening sequence is deleted in the presence of the recombinase, wherein said cell is a embryonic stem cell or an adult stem non-human cell,
(b) selecting those cells wherein the DNA construct has recombined with the GDNF locus,
(c) developing from said cell a viable non-human animal,
(d) breeding the non-human animals obtained in step (c) with a strain from the same non-human species which contains into their genome a second nucleic DNA construct coding for a recombinase which is operably linked to a tissue-specific promoter and
(e) selecting those animals from the offspring which contain both the recombined GDNF locus and the second nucleic acid molecule.

In a preferred embodiment, the animal is a mammalian animal. In another preferred embodiment, the recombinase is a Cre recombinase. In yet another preferred embodiment, the tissue-specific promoter is a neural specific promoter, more preferably the GFAP promoter.

In another aspect, the invention provides transgenic non-human animals which are produced by the methods previously described. The transgenic animals will then contain a modified allele of the GDNF gene wherein an essential part of it is flanked by recombinase target sites and a transgene which encodes for a recombinase which recognizes the target sites in the GDNF allele and which is under the control of a tissue-specific promoter. The presence of a recombinase under the control of a tissue-specific promoter will result in the expression of the recombinase in those tissue of the animal wherein the promoter is usually active, thus leading to the excision of the essential part of the GDNF gene and the inactivation of the gene. If both alleles of the transgenic animal contain the recombinase target sites, the resulting animal will thus carry a homozygous inactivation of the GDNF gene, resulting in that both copies of the gene are inactivated. All other remaining tissues of the non-human animal wherein the promoter is not expressed will show normal expression levels of GDNF.

In another aspect, the invention provides an organ, tissue or cell isolated from the non-human transgenic animals of the invention. In a preferred embodiment, the organ is the brain.

As previously mentioned, the authors of the present invention have observed that animals carrying both transgenes and wherein the recombinase is expressed in the brain under the control of the neural cell specific promoter do not show any changes in TH+ neuron counts either in *GDNF* heterozygous mice or in the embryonic *GDNF* knockouts restricted to GFAP-producing cells. Surprisingly, in the early embryonic GDNF knock out heterozygotes, it was observed an increase of brain *GDNF* mRNA content. Without wishing to be bound by any particular theory, it is thought that the lack of GDNF in glial cells (and in neurons derived from radial glia) is compensated by over-production of GNDF in other cell types.

Accordingly, in another aspect, the invention provides the use of a non-human animal with a tissue-specific deletion of the GDNF gene for the identification of compounds which promote the up-regulation of GDNF mRNA in response to down-regulation of GDNF in the neighbouring glial cells due to the disruption of one or two copies of the gene in said neighbouring tissues. According to these methods, the animal is contacted with a compound to be tested and the effects of said compound on GDNF levels in the nervous system are measured so that if the compound leads to an increase in the expression level of GDNF in the brain, then the compound is selected as candidate for use for the treatment of diseases wherein GDNF levels are abnormally decreased.

In another aspect, the invention provides the use of a non-human animal with a tissue-specific GDNF knock out for the identification of compounds which prevent up-regulation of GDNF in the brain in response to the knock out of said gene in the neighbouring glial cells. According to these methods, an animal is contacted with a test compound and those animals which do not show up-regulation of GDNF in the brain are selected for the use of conditions wherein GDNF levels are abnormally increased.

In another aspect, the invention provides the use of a non-human animal with a tissue-specific GDNF knock out for the study of the role of GDNF in embryonic development.

The following examples illustrate the invention and are not to be construed as limiting the scope of the invention.

### EXAMPLES

### EXAMPLE 1

### Generation of Animal Models

Animals were housed at a regulated temperature (22 °C) in a 12 h light/dark cycle. All experiments were performed in accordance with institutional guidelines approved by the ethical committee in the "Hospital Universitario Virgen del Rocio". To generate the GDNF conditional animals, we engineered a targeting construct that could be homologously recombined into the third coding exon of the GDNF locus. The targeting construct contained the GDNF E3 flanked by loxP sites (*GDNF^{F}*) and neomycin resistance marker. For homologous recombination, we used 129Sv-derived R1 ES cells. Southern blotting using a DNA 3' probe identified the recombined allele. *GDNF^{F}* mice were further propagated and used for experiments on a mixed genetic background (129/SvJ:C57BL/6). Routine genotyping to detect the wild-type or floxed allele was performed using PCR. Floxed PCR was performed with primers loxP15 (5'-TCACGTGTCTATGTGCTAAA-3') and loxP13 (5'-AATGATCATTTCGGGCAGTC-3'). To obtain the two experimental models used in this study, *GDNF*/*LacZ* animals (*GDNF^{Z}*/+; Sanchez et al., 1996, Nature, 382:70-73) were either matted with *Cre-Esr1*/+ (Hayashi and McMahon, 2002, Dev. Biol., 244:305-318) or *GFAP-Cre*/*+* (Zhuo et al., 2001, Genesis, 31:85-94) mice to obtain a F1 *GDNF^{Z}*/+*; Cre*/+ progeny. F1 animals were matted with *GDNF^{F}*/+ mice to generate control (*GDNF*^{*+*/}*^{F};* +/+) and experimental littermate (*GDNF*^{*Z*/}*^{F}; Cre*/*+)* mice. In the Cre-Esr1 animal model, 2 months old animals were intraperitoneally injected with TM (Sigma; 0.2 mg/g/day) during four consecutive days. TM was prepared and used as previously described (Guo et al., 2002, Genesis, 32:8-18). *GDNF*^{*Z*/}*^{F}; Cre-Esr1*/+ without TM treatment was used as a control when indicated. To test successful conditional deletion of GDNF *in vivo*, excised PCR was used with primers loxP15 and loxP23 (5'-GAACTCCAGGTAAATAATCC-3'). To verify TM effect and the proper excision at the *GDNF^{F}* locus, *GDN*^{*+*/}*^{F}; Cre-Esr1*/+ mice treated with TM were matted with wild-type animals. Heterozygous GDNF-null mice in the progeny were crossed and, as expected, the resulting *GDNF*^{*-*/*-*} offspring died during the first postnatal day and presented renal agenesis.

### DNA Extraction from Brain Paraffin Slices.

Striatal 20 µm paraffin embedded slices were de-paraffined twice with xylene and hydrated by washing with solutions containing decreasing ethanol concentration. Four slices were digested in 200 µl of directPCR reagent (Viagen) containing proteinase K (15 µg/ml) at 55 °C for 2 hours. Proteinase K was inactivated by heating samples at 85 °C for 2 hours. 1 µl from the resulting DNA solution was used for PCR analysis.

### Quantitative RT-PCR

To determine *GDNF*, *Th*, *Chat*, and *Gad1* mRNA levels brain RNA was extracted using TRIzol reagent (Invitrogen) in a homogenizer (Omni 2000). RNA samples (5 µg) were treated with DNase-RNase free (GE Healthcare) and copied to cDNA using SuperScriptII reverse transcriptase (Invitrogen) in a final volume of 20 µl. Real time PCR was performed in an ABI Prism 7500 Sequence Detection System (Applied-Biosystems) using SYBR Green PCR Master mix (Applied-Biosystems) and the thermocycler conditions recommended by the manufacturer. PCR reactions were performed in duplicates in a total volume of 25 µl containing 1 µl of the RT reaction. In each sample, *Actb* and *Ppia* RNA levels were estimated to normalize for RNA input amounts. Relative quantifications using both housekeeping genes produced equivalent results. To normalize mRNA knockout mice levels to control samples, an average *Ct* of the control samples was calculated and all the samples in the experiment were processed as relatives to this average *Ct*. Primers were designed using the Primer Express software (Applied-Biosystems). The following primers were used: *β-actin* (*Actb*), 5'-GGCCCAGAGCAAGAGAGGTA-3' and 5'-CATGTCGTCCCAGTTGGTAACA-3'; *cyclophilin A* (*Ppia*), 5'-ATGGCAAATGCTGGACCAAA-3'and 5'-TGCCATCCAGCCATTCAGT-3'; *tyrosine hydroxylase* (*Th*), 5'-GGCTTCTCTGACCAGGCGTAT-3' and 5'-GCTCACCCTGCTTGTATTGGA-3'; *choline acetyltransferase* (*Chat*), and 5'-GGAGCGAATCGTTGGTATGAC-3' and 5'-ATCTCGGCCCACCACAAA-3'; *glutamic acid decarboxylase* (*Gad1*), 5'-ACTCCTCAACTATGTCCGCAAGA-3' and 5'-TCCAAATTAAAGCCTTCCATGC-3'; *GDNF,* 5'-GGATGGGATTCGGGCCACT-3' and 5'-AGCCACGACATCCCATAACTTC-3'.

### Tissue Preparation and Histochemistry

Animals were anesthetized and fixed with 4% paraformaldehyde. The brain and the carotid bifurcation were extracted and paraffin embedded. Coronal brain sections (20 µm) from adult mice (9 months of age) were used for tyrosine hydroxylase (Th), NeuN-Th immunohistochemistry, or Nissl staining (0.5% cresyl violet) in quantitative and morphometric studies. Envision+ kit (DAKO) was used for immunohistochemistry according to the recommended protocol. The antibodies and the dilution factor used were as follow: anti-Th polyclonal antibody, Pel-Freez, 1:1000; anti-NeuN monoclonal antibody, Chemicon, 1:200. For double labeling with anti-Th and anti-NeuN antibodies we incubated the slices with antibodies against mouse antibodies and the signal was developed with DAB (DAKO). To increase contrast a DAB enhancer (DAKO) was used. Antibodies against rabbit antibodies were added in a second step and the reaction developed with AEC subtract (DAKO). β-galactosidase staining in 50 µm brain slices was performed as described previously (Sanchez et al., 1996, Nature, 382:70-73; Villadiego et al., 2005, J.Neurosci., 25:4091-4098).

### Stereological Cell Counts

Counts of TH-, NeuN-immunoreactive, and Nissl-positive neurons were performed in 20 µm coronal microtome sections spaced 40 µm throughout the extent of NA, SN, VTA and LC. The number of cells was estimated by systematic random sampling using a 128,345.4 µm3 optical dissector (West, 1993) excluding neurons in the superficial planes of sections. The volume of the different brain regions was estimated according to the principle of Cavalieri (Cavalieri, 1966, Torino: Unione Tipografica Editrice). Stereological measurements were done using the C.A.S.T. Grid System (Olympus). Striatal TH+ fiber density was quantified in a 20 µm slice at Bregma 0.86 (Franklin and Paxinos, 1996, The Mouse Brain in Stereotaxic Coordinates, New York: Academic Press). Striatum was divided into dorsal and ventral areas and 10-20 random 3,565.2 µm2 dissectors per hemisphere were counted using the C.A.S.T. Grid System. CB TH+ cell counting was performed across complete CB parenchyma.

| **Table 1. Catecholaminergic cell death in adult conditional GDNF-null mice** | | | | | | |
|---|---|---|---|---|---|---|
| | | ***GDNF*^{*F*/+}** **(TM+)** | ***GDNF*^{*F*/}*^{LacZ};*** ***Cre-Esr1*(TM-)** | ***GDNF*^{*F*/}*^{LacZ};*** ***Cre-Esr1* (TM+)** | ***GDNF*^{*F*/+}** | ***GDNF*^{*F*/}*^{LacZ};*** ***GFAP-Cre*** |
| **SN** | **TH+** | 4787±480 | 4931±865 | 2029±469**/* | 4660±553 | 3870±326 |
| | | (*n* = 8) | (*n* = 6) | (*n* = 6) | (*n* = 8) | (*n* = 5) |
| | **NeuN+** | 11289±510^{$} | | 8356±720** | | |
| | | (*n* = 6) | | (*n* = 6) | | |
| **VTA** | **TH+** | 2561±427 | 2919±238 | 872±381*/** | 2643±376 | 2576±677 |
| | | (*n* = 6) | (*n* = 6) | (*n* = 6) | (*n* = 7) | (*n* = 4) |
| | **NeuN+** | 5855±421^{$} | | 2156±586** | | |
| | | (*n* = 6) | | (*n* = 6) | | |
| **LC** | **TH+** | 873±131 | 850±301 | 5±3**/** | 1119±159 | 1150±305 |
| | | (*n* = 5) | (*n* = 5) | (*n* = 6) | (*n* = 5) | (*n* = 3) |
| | **Nissl+** | 2489±363^{$} | | 1537±169* | | |
| | | (*n* = 7) | | (*n* = 6) | | |
| **NA** | **TH+** | 264±99^{$} | | 268±109 | | |
| | | (*n* = 8) | | (*n* = 6) | | |
| **dST** | **TH+** | 21.4±1.6^{$} | | 15.4±1.4* | | |
| | | (*n* = 12) | | (*n* = 6) | | |
| **vST** | **TH+** | 26.9±2.2^{$} | | 17.1±1.7** | | |
| | | (*n* = 9) | | (*n* = 6) | | |
| **CB** | **TH+** | + 372.3±14.2^{$} | | 167.2±80.3* | | |
| | | (*n* = 4) | | (*n* = 3) | | |
| **SCG** | **Volume** | 0.257±0.008^{$} | | 0.174±0.010** | | |
| | | (*n* = 4) | | (*n* = 3) | | |
| ^{$} *GDNF*^{*F*/+} treated with TM and *GDNF*^{*F*/}*^{LacZ}; Cre-Esr1* control samples were counted together. Asterisks indicated significant differences from *GDNF*^{*F*/+} (TM+) (*/), *GDNF*^{*F*/}*^{LacZ}; Cre-Esr1* (TM-) (/*), or pooled groups (*). *: *p* < 0.05; **: *p* < 0.01. | | | | | | |

### EXAMPLE 2

### Conditional GDNF knock-out mice generation

To modify the GDNF locus, the 15th chromosome genomic region surrounding the exon 3 of GDNF was cloned. The cloned region contained 4.5 kb of intron 2 and 4.2 kb downstream of exon 3. The cloning was performed using 4 radioactive probes homologous to the genomic region of interest. The probes were hybridised with a 129sv lambda phage genomic library. The identified DNA fragment was subcloned into pBS using *Xho*I. The first loxP sequence was cloned in the *Xba*I site of the resulting DNA fragment containing the *Xho*I upstream sequence. The neomycin marker and the second loxP sequences were cloned in the *Nco*I site of the *Xho*I downstream sequence. Both fragments were cloned in the same plasmid, made linear by *Not*I enzymatic digestion and 10 µg of linear DNA were electroporated into mouse embryonic stem cells. Geneticine (G-418) was added to the culture the day after the electroporation and the culture medium was changed every day for ten days. 334 geneticine resistant clones were picked, cultured, and amplified to produce two culture dishes per clone. One culture was frozen and the other used for DNA extraction. The genomic DNA obtained was digested with *Bam*HI, migrated in a 0.6 % agarose gel and transferred to a positive charged membrane. The membrane was hybridized with an external 3' probe (a *Eco*RV/*Bam*HI fragment from the GDNF genomic region cloned, Figure 1A). The clones harbouring the targeting construct integrated in the GDNF locus (Figure 1A-B) were analyzed for PCR regarding the integration of the 5' loxP sequence (Figure 1A, C). The positive clones (3) were electroporated with a plasmid containing a puromycin maker and 5 puromycin resistant clones were picked. Deletion of GDNF exon 3 was checked by Southern blot and PCR. The embryonic stem cells harbouring the targeted construction integrated by homologous recombination were injected into blastocysts of C57BL/6J and Swiss females. The resulting chimeric mice were crossed with C57BL/6J females to generate the *GDNF*^{*F*/+} animals.

### EXAMPLE 3

### Analysis of a mice model with inactivation of GDNF during adulthood

The floxed animals were interbred with heterozygous null-*GDNF* mice (*GDNF*^{*+*/*Z*}) carrying the *Cre-Esr1* transgene in which ubiquitously expressed Cre recombinase can be activated by tamoxifen (TM) (Hayashi and McMahon, 2002, Dev.Biol., 244:305-318). TM was administered at two months of age and appearance of the *GDNF*-null allele in the germline was tested (see Example 1). Animals were sacrificed either at five months for mRNA analysis or at nine months for histological studies (Fig. 2A). Confirmation for the excision of the floxed allele in adulthood was done by PCR of striatal DNA using specific probes (Fig. 2B).

*GDNF* mRNA expression was evaluated by quantitative RT-PCR performed on whole brain extracts (Fig. 2C). In mice containing the *Cre-Esr1* transgene, embryonic *GDNF* heterozygosity resulted in a mild, non-significant decrease of total brain *GDNF* mRNA content that was similar in *GDNF*^{+/*Z*} and *GDN*^{*F*/*Z*} strains. This indicates that the floxed *GDNF* allele was transcribed with similar efficiency as in the wild type. Administration of TM had no effect on wild type animals, but in *GDN*^{*F*/*Z*} mice induced a marked (∼60%, p<0.01) reduction of *GDNF* mRNA. Although brain total mRNA content provides only a rough estimate of GDNF expression levels, these data demonstrate that TM administration to the conditional GDNF knockout mice leads to a large decrease of GDNF mRNA, possibly affecting the survival of adult neurons with a neurotrophic dependence on this factor.

To evaluate the impact of TM treatment and subsequent GDNF depletion on neuronal viability, stereological cell counts in several brain regions were performed, with emphasis on those areas containing catecholaminergic neurons whose vulnerability to neurotoxins is prevented by GDNF. The most representative data obtained from these experiments (animals sacrificed seven months after TM treatment) are illustrated in Fig. 3 and 4 and a quantitative summary is given in Table 1. *GDNF* heterozygosity (*GDNF*^{*F*/}*^{Z}, Cre-Esr1* strain), that as described above resulted in a minor decrease of total brain GDNF mRNA levels, had no effect on the number of TH+ neurons in the SN, VTA or LC compared with controls (*GDNF*^{+/*F*} strain). Deletion of the floxed allele with TM gave rise, however, to a marked (∼60%) reduction in the number of TH+ cells as well as a significant decrease in the total number of neurons both in the SN and VTA (Fig. 3A-D). Consistent with an earlier developmental study (Kholodilov et al., 2004, J.Neurosci., 24:3136-3146), the survival of neurons in the VTA appeared to rely more on GDNF than did SN cells. These effects were paralleled by a reduction of the density of TH+ striatal nerve fibers, particularly in the ventral region (Table 1). Neuronal death was even more dramatic in the LC, since evidence of TH+ neurons in this nucleus almost disappeared (Fig. 4A-F, Table 1). A similar profile of catecholaminergic cell death, although quantitatively less marked, was observed in animals only four months after TM treatment, thus indicating that brain depletion of GDNF initiates a process of progressive neuronal degeneration (data not shown). The loss of mesencephalic catecholaminergic neurons in GDNF-deprived animals was paralleled by a decrease of TH+ cells in structures of the peripheral nervous system such as the carotid body (CB; Fig. 4G, H) or the superior cervical ganglion (SCG) (Table 1).

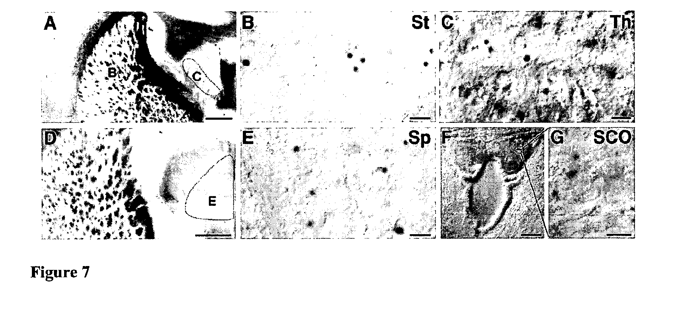

Neuronal loss observed in conditional GDNF-depleted mice was not general but restricted to the above indicated brain areas. For example, the number of TH+ cells in the nucleus arcuatus, a component of the diencephalic dopaminergic system, was unchanged (Fig. 5A,B; Table 1) and no apparent gross histological alterations were observed in the hippocampus or in neocortical regions (Fig. 5C-F). GDNF depletion was also accompanied by a selective decrease of brain *Th* mRNA content, leaving choline acetyltransferase (*Chat*) and glutamate decarboxylase (*Gad1*) mRNA levels unaltered (Fig. 6). These data indicate that GDNF deletion in adulthood leads to extensive central and peripheral catecholaminergic cell death in specific brain areas, without apparent major damage to cholinergic or gabaergic neurons. Given the striking dependence on GDNF of mesencephalic dopaminergic and noradrenergic neuronal survival, we searched for the location of putative targets constitutively expressing high levels of GDNF and therefore critical for the trophic maintenance of the cells. To study precisely the localization of GDNF-producing cells in adult brain, heterozygous *GDNF*/*LacZ* mice were used, in which GDNF+ cells can be labelled by the characteristic blue X-gal staining (Sanchez et al., 1996, Nature, 382:70-73). As discussed previously, this technique detects only cells with high levels of GDNF expression and is free of experimental artifacts frequently observed with other methods (immunocytochemistry or in situ hybridization) due to the low levels of GDNF expression in most brain areas (Villadiego et al., 2005, J.Neurosci., 25:4091-4098). After systematic analysis of the brain, X-gal+ deposits were consistently found in cells distributed throughout the striatum, in the anteroventral (particularly the ventrolateral region) and anteromedial nuclei of the thalamus as well as in the septum (mainly in the mediolateral region) (Fig. 7A-E). These structures are innervated by projections from catecholaminergic neurons of the SN, VTA and LC (Lindvall and Stenevi, 1978, Cell Tissue Res., 190:383-407; Bjorklund and Hokfelt, 1984, Handbook of Chemical Neuroanatomy: Classica Neurotrasmitters in the CNS Part I (Amsterdam, Elsevier)). Interestingly, a high level of GDNF expression was also observed in the subcommissural organ (SCO) (Fig. 7F,G), an ependymal secretory gland located at the roof of the third ventricle that participates in cerebrospinal fluid (CSF) homeostasis (Galarza, 2002, Neurosurg. Rev., 25:205-215).

### EXAMPLE 4

### Analysis of a mice model with inactivation of GDNF during embryogenesis

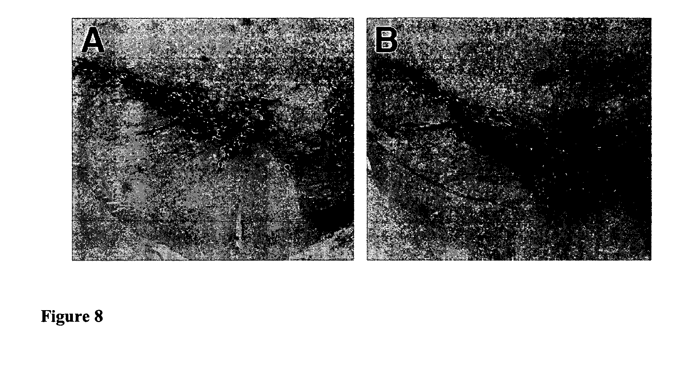

The floxed animals were interbred with heterozygous null-*GDNF* mice (*GDNF*^{+/*Z*}) carrying the *GFAP-Cre* transgene, in which the *GDNF^{F}* allele was excised during embryonic life. (Zhuo et al., 2001, Genesis, 31:85-94). Animals were sacrificed either at five months for mRNA analysis or at nine months for histological studies (Fig. 2A). Confirmation for the excision of the floxed allele in adulthood was done by PCR of striatal DNA using specific probes (Fig. 2E).
*GDNF* mRNA expression in the various mouse strains studied was evaluated by quantitative RT-PCR performed on whole brain extracts (Fig. 2F). In mice containing the *GFAP-Cre* transgene, brain *GDNF* mRNA levels were ∼50% higher than in controls, thus suggesting that embryonic *GDNF* deletion in GFAP-expressing cells resulted in a compensatory up-regulation of *GDNF* in other cell types. In full agreement with the *GDNF* mRNA measurements described above, the number of TH+ cells in the SN, VTA or LC of *GDNF*^{*F*/}*^{Z}; GFAP-Cre* mice was unchanged compared to respective control values (Fig. 8 A,B; Table 1).

## Claims

1. A DNA construct comprising at least a region of the GDNF gene flanked by recombinase target sites, wherein said region of the GDNF gene is necessary for the expression of a functional GDNF protein and wherein said recombinase target sites are oriented so that the intervening sequence is deleted in the presence of the recombinase.

2. A DNA construct as defined in claim 1 wherein said region of the GDNF gene comprises at least one exon of said gene.

3. A DNA construct as defined in claim 2 wherein the exon of the GDNF gene comprised in the construct is exon 3.

4. A DNA construct as defined in any one of claims 1 to 3 wherein the recombinase target sites are loxP sites.

5. A DNA construct according to any one of claims 1-4 further comprising a resistance marker.

6. A vector comprising a DNA construct according to any of claims 1-5.

7. A host cell comprising a vector as defined in claim 6 or a DNA construct as defined in any one of claims 1 to 5.

8. A cell obtainable by homologous recombination between the DNA construct as defined in any one of claims 1 to 5 and the GDNF locus.

9. The cell of claims 8 or 9, wherein said cell is eukaryotic.

10. The cell of claim 9, wherein said vertebrate cell is a cell of xenopus, zebra fish or a mammalian cell.

11. The cell of claim 10, wherein said mammalian cell is an embryonic stem cell, an embryonic germ cell, an adult stem cell or a somatic cell.

12. A method for the generation of a transgenic non-human animal, comprising the steps of propagating the cell of claim 11 and developing from said cell a viable organism, wherein said cell is non-human.

13. A non-human animal obtainable by the method of claim 12.

14. A method for the generation of a transgenic non-human animal, comprising the steps of breeding animals as defined in claim 13 and selecting those animals wherein both loci of the GDNF gene have recombined with the targeting construct.

15. A non-human animal obtainable by the method of claim 14.

16. A method for obtaining a cell or a non-human animal carrying an inactive GDNF gene comprising the steps of
(a) contacting a cell as defined in any one of claims 8 to 11 or an animal as defined in claim 15 with a compound which is capable of inducing the expression of a recombinase specific for the target sites present in the GDNF and
(b) selecting those cells or animals wherein the recombinase has catalysed the excision of the functional part of the GDNF locus.

17. A method as defined in claim 16 wherein the compound capable of increasing the activity of a recombinase specific for the target sites is a nucleic acid molecule which comprises region coding for a recombinase.

18. A method as defined in claim 17 wherein said compound comprising a nucleic acid coding for a recombinase is an adenovirus.

19. A cell or a non-human animal obtainable by a method as defined in any one of claims 16 to 18.

20. A method for the generation of a conditional knock-out cell in the GDNF gene comprising the steps of:
(a) introducing a nucleic acid molecule comprising the DNA construct of any one of claims 1 to 5 into a cell which expresses a recombinase whose expression and/or activity can be regulated by an external signal and
(b) selecting cells which wherein the DNA construct introduced in step (a) has recombined with the GDNF locus.

21. A method for the generation of a conditional knock-out cell in the GDNF gene, comprising the steps of:
(a) introducing into a cell a first nucleic acid molecule comprising the DNA construct of any one of claims 1 to 5,
(b) selecting those cells wherein the DNA construct has recombined with the GDNF locus,
(c) introducing into the cells selected in step (b) a second nucleic acid molecule coding for a recombinase whose expression and/or activity can be regulated by an external signal and
(d) selecting those cells which have integrated into their genome the second nucleic acid molecule.

22. A method for the generation of a conditional knock-out cell in the GDNF gene, comprising the steps of:
(a) introducing into a cell a first nucleic acid molecule comprising the DNA construct of any one of claims 1 to 5 and a second nucleic acid molecule coding for a recombinase whose expression and/or activity can be regulated by an external signal.
(b) selecting those cells wherein the DNA construct of the first nucleic acid molecule has recombined with the GDNF locus and which have incorporated into their genome said second nucleic acid molecules.

23. A method as defined in claims 21 or 22 wherein the first nucleic acid molecule and the second nucleic acid molecules contain different selection markers.

24. A method as defined in any one of claims 20 to 23 wherein the nucleic acid coding for the recombinase is operably linked to an inducible promoter.

25. A method as defined in any one of claims 20 to 23 wherein the recombinase is coupled in frame to an activable NLS.

26. A method as defined in any one of claims 25 wherein the activable NLS is the ligand binding domain of the estrogen receptor.

27. A method as defined in claims 25 or 26 wherein the gene coding for the recombinase coupled to an activable NLS is operably linked to a constitutive promoter.

28. A method as defined in any one of claims 20 to 27 wherein the recombinase is Cre recombinase.

29. A cell generated by the method of any one of claims 20-28.

30. The cell of claim 29, wherein said cell is eukaryotic

31. The cell of claim 30 wherein said mammalian cell is an embryonic stem cell, an embryonic germ cell, an adult stem cell or a somatic cell.

32. A method of obtaining a cell carrying an inactive GDNF gene comprising the steps of
(a) contacting a cell as defined in any one of claims 29 to 31 with a compound which is capable of inducing the expression and/or activity of the recombinase and
(b) selecting those cells wherein the recombinase has catalysed the excision of a functional part of the GDNF locus.

33. A method as defined in claim 32 wherein the compound which is capable of inducing the expression and/or activity of the recombinase is selected from the group of an inducer of the promoter which controls expression of the recombinase or an inducer of an activable NLS.

34. A method for the generation of a transgenic non-human animal, comprising the steps of
(a) introducing into a cell a nucleic acid molecule comprising a DNA construct of any one of claims 1 to 5 wherein said cell is a embryonic stem cell or an adult stem non-human cell,
(b) selecting those cells wherein the DNA construct has recombined with the GDNF locus,
(c) developing from said cell a viable non-human animal,
(d) breeding the non-human animals obtained in step (c) with a strain from the same non-human species which contains into their genome a second nucleic DNA construct coding for a recombinase whose expression and/or activity can be regulated by an external signal.
(e) selecting those animals from the offspring which contain both the first nucleic acid molecule and the second nucleic acid molecule.

35. A method for the generation of a transgenic non-human animal, comprising the steps of
(a) introducing into a cell a first nucleic acid molecule comprising the DNA construct of any one of claims 1 to 5,
(b) selecting those cells wherein the DNA construct has recombined with the GDNF locus,
(c) introducing into the cells selected in step (b) a second nucleic acid molecule comprising the DNA construct of a second nucleic acid coding for a recombinase whose expression and/or activity can be regulated by an external signal,
(d) selecting cells which have integrated the second nucleic acid molecule and
(e) developing from said cell a viable non-human animal,

36. The method of claim 34 or 35, wherein said animal is a mammal.

37. A method as defined in any one of claims 34 to 36 wherein the nucleic acid coding for the recombinase is operably linked to an inducible promoter.

38. A method as defined in any of claims 34 to 36 wherein the recombinase is coupled in frame to an activable NLS

39. A method as defined in claim 38 wherein the activable NLS comprises the ligand binding domain of the estrogen receptor.

40. A method as defined in any one of claims 34 to 39 wherein the recombinase is Cre recombinase.

41. A transgenic non-human animal produced by the method of claims 34 or 40.

42. A method for the generation of a transgenic non-human animal, comprising the steps of breeding animals as defined in claim 41 and selecting those animals wherein both loci of the GDNF gene have recombined with the targeting construct.

43. A method for obtaining a non-human animal deficient in at least one GDNF allele which comprises contacting an animal as defined in claim 41 or 42 with a compound which is able to induce the expression or activity of the recombinase so that the recombinase-mediated excision of a functional part of the GDNF locus takes place.

44. A method as defined in claim 43 wherein the compound activates transcription of the recombinase by activation of the inducible promoter.

45. A method as defined in claim 43 wherein the compound activates translocation of the recombinase to the nucleus by activation of the activable NLS.

46. A method as defined in claim 45 wherein the activable NLS is the ligand binding domain of the estrogen receptor and the ligand able to activate said NLS is a estrogen.

47. A method as defined in claim 46 wherein the estrogen is tamoxifen.

48. A non-human animal obtainable by the method of any one of claims 43 to 47.

49. An organ, tissue or cell isolated from a non-embryonic non-human animal according to claims 19 or 48.

50. An organ as defined in claim 49 wherein the organ is the brain.

51. Method for the identification of compounds suitable for the treatment or for the prevention of a neurodegenerative disease comprising:
(a) contacting a non-human animal as defined in claims 19 or 48 with a test compound and
(b) selecting those compounds which prevent or ameliorate neuronal degeneration.

52. Use of a non-human animal as defined in claims 19 or 48 for the study of the mechanisms involved in neuronal degeneration induced by GDNF deficiency.

53. Use of a non-human animal as defined in claims 19 or 48 for the identification of GDNF agonists.

54. Use of a non-human animal as defined in claims 19 or 48 for the optimization of the delivery into the animal of GDNF or agonists thereof.

55. A method for the generation of a conditional knock-out cell in the GDNF gene comprising the steps of:
(a) introducing a nucleic acid molecule comprising the DNA construct of any one of claims 1 to 5 into a cell which contains within its genome a gene coding for a recombinase operably linked to a tissue-specific promoter.
(b) selecting cells wherein the DNA construct has recombined with the GDNF locus.

56. A method for the generation of a conditional knock-out cell in the GDNF gene, comprising the steps of:
(a) introducing into a cell a first nucleic acid molecule comprising the DNA construct of any one of claims 1 to 5,
(b) selecting cells wherein the DNA construct has recombined with the GDNF locus,
(c) introducing into the cells selected in step (b) a second nucleic acid molecule comprising a gene coding for a recombinase which is operably linked to a tissue specific promoter and
(d) selecting cells which have integrated into their genome the second nucleic acid molecule.

57. A method for the generation of a conditional knock-out cell in the GDNF gene, comprising the steps of:
(c) introducing into a cell a first nucleic acid molecule comprising the DNA construct of any one of claims 1 to 5 and a second nucleic acid molecule coding for a recombinase which is operably linked to a tissue-specific promoter and
(d) selecting those cells wherein the DNA construct has recombined with the GDNF locus and which have incorporated into their genome said second nucleic acid molecules.

58. A method as defined in claims 55 or 56 wherein the first nucleic acid molecule and the second nucleic acid molecule contain a selection marker which is different between the first and second nucleic acid molecules.

59. A method as defined in any one of claims 54 to 58 wherein the tissue-specific promoter is a neural cell specific promoter.

60. A method as defined in claim 59 wherein the neural cell specific promoter is the GFAP promoter.

61. A method as defined in any one of claims 54 to 60 wherein the recombinase is Cre recombinase.

62. A cell generated by the method of any one of claims 54-61.

63. The cell of claim 62, wherein said cell is eukaryotic.

64. The cell of claim 63, wherein said vertebrate cell is a cell of xenopus, zebra fish or a mammalian cell.

65. The cell of claim 64, wherein said mammalian cell is an embryonic stem cell, an adult stem cell or a somatic cell.

66. A method for the generation of transgenic non-human animal, comprising the steps of propagating the cell of claim 65 and developing from said cell a viable organism, wherein said cell is non-human.

67. A method for the generation of a transgenic non-human animal, comprising the steps of
(a) introducing into a cell a first nucleic acid molecule comprising the DNA construct of any one of claims 1 to 5 wherein said cell is a embryonic stem cell or an adult stem non-human cell,
(b) selecting those cells wherein the DNA construct has recombined with the GDNF locus,
(c) developing from said cell a viable non-human animal,
(d) breeding the non-human animals obtained in step (c) with a strain from the same non-human species which contains into their genome a second nucleic DNA construct coding for a recombinase which is operably linked to a tissue-specific promoter.
(e) selecting those animals from the offspring which contain both the first nucleic acid molecule and the second nucleic acid molecule.

68. The method of claim 67, wherein said animal is a mammalian animal.

69. A method as defined in any one of claims 67 or 68 wherein the recombinase is Cre recombinase.

70. A method as defined in any one of claims 67 to 69 wherein the tissue-specific promoter is a neural cell specific promoter.

71. A method as defined in claim 70 wherein the neural cell specific promoter is the GFAP promoter.

72. A transgenic non-human animal produced by any one the method of any one of claims 66 to 71.

73. An organ, tissue or cell isolated from a non-human animal according to claim 72.

74. An organ as defined in claim 73 wherein the organ is the brain.

75. Use of a non-human animal as defined in claim 72 for the identification of compounds which promote the GDNF mRNA up-regulation in response to a down-regulation of GDNF in the neighboring cells.

76. Use of a non-human animal as defined in claim 72 for the identification of compounds which prevent the up-regulation of GDNF mRNA.

77. Use of a non-human animal as defined in claim 72 for the study of the role of GDNF in embryonic development.
